# EUROPEAN PATENT APPLICATION

(11) **EP 4 837 592 A1**
(43) Date of publication of application: **11.11.2026**
(21) Application number: 26176990.5
(22) Date of filing: 06.05.2026
(51) Int. Cl.: A61K 47/69, A61K 9/51, A61K 47/54, A61K 47/61, A61P 37/00, C12N 15/11, C12N 15/88

(54) **MULTI-VALENT LSEC TARGETING MRNA LIPID NANOPARTICLE AND ITS APPLICATION**

(30) Priority: 07.05.2025 CN 202510584392; 23.05.2025 CN 202510674912; 01.07.2025 CN 202510900375
(71) Applicant: Zhejiang MaRiNA BioTherapeutics Co., Ltd., Hangzhou, Zhejiang (CN)
(72) Inventor: XIA, Tian, Hangzhou (CN); SU, Jeffrey Yanjing, Hangzhou (CN)
(74) Representative: Loo, Chi Ching

(57) **Abstract**

The present invention discloses a lipid nanoparticle (LNP) and its application, comprising cationic ionisable lipids, auxiliary lipids, ligand-modified PEGylated lipids, and cholesterol. The auxiliary lipids and ligand-modified PEGylated lipids target receptors on the surface of liver sinusoidal endothelial cells (LSECs). The LNPs enhance LSEC-specific uptake through a targeted design involving the lipid-specific interaction pathway.

## Description

### CROSS-REFERENCE TO RELATED APPLICATIONS

This application claims priority from an earlier Chinese application, application number: 202510584392.9, filed on 07 May, 2025; 202510674912.5, filed on 23 May, 2025 and 202510900375.1 filed on 03 July,2025, the entirety of said application forms part of this invention.

### SEQUENCE LISTING

A sequence listing is being submitted with this application. This sequence listing is submitted as the file name "MULTI-VALENT LSEC TARGETING mRNA LIPID NANOPARTICLE AND ITS APPLICATION" with file size of 192,512 bytes and a date of creation of February 09,2026. This document is hereby incorporated by reference in its entirety.

### BACKGROUND OF THE INVENTION

### Field of the Invention

The present invention belongs to the field of drug delivery, specifically relating to a lipid nanoparticle and its application.

### Description of the Related Art

Beyond current treatments using anti-inflammatory, immunosuppressive, targeted monoclonal antibodies, or immunomodulatory methods, the unmet need for developing new treatments for autoimmune and allergic diseases persists. While most of these therapies provide symptomatic relief and temporary reduction in disease activity, they do not offer the prospect of long-term suppression of chronic disease activity or a cure. However, there is an increasing awareness of the power of regulatory T cell (Treg) biology, highlighting the importance of providing antigen-specific immune tolerance for autoimmune diseases (such as rheumatoid arthritis, lupus, type I diabetes, etc.) and allergic diseases (such as food allergies, anaphylaxis, asthma, etc.). Inducing antigen-specific tolerance is a method using biodegradable nanoparticles to initiate and maintain immunomodulatory responses, based on the ability of these carriers to encapsulate disease-related antigens which are delivered to antigen-presenting cells (APCs), thereby inducing antigen-specific tolerance.

The liver's tolerance is well-recognized, as this organ significantly prevents immune responses to exogenous food antigens from the gastrointestinal tract and portal venous system, while also promoting the persistence of tumour metastasis to this organ. Furthermore, the liver enjoys immune privilege during organ transplantation, requiring less immunosuppressive therapy compared to kidney or heart transplants. Research indicates that simultaneous transplantation of kidneys or hearts with the liver is associated with a lower incidence of immune rejection compared to isolated organ transplantation.

The liver's immunosuppressive function is partly attributable to its unique antigen-presenting cell (APC) system, comprising naturally tolerant APCs such as Kupffer cells (KC), dendritic cells (DC), and liver sinusoidal endothelial cells (LSECs).These tolerogenic APCs form an integral part of the hepatic reticuloendothelial system, which possesses key functions in clearing foreign particles, degradation products, and toxins from sinusoidal blood through phagocytic uptake and endocytic processing. Moreover, professional phagocytes (KCs and DCs) preferentially eliminate micrometre-scale particulate matter in the circulation via phagocytosis, whilst LSECs are more adept at clearing soluble macromolecules and nanoparticles within the 200 nm range through clathrin-mediated endocytosis. From an immunoregulatory perspective, LSECs play a pivotal role in inducing immunosuppression within CD8+ and CD4+ populations through the generation of antigen-specific Tregs, TGF-β production, and upregulation of the programmed cell death protein 1 (PD-1) receptor ligand (PD-L1). Consequently, the capacity of LSECs to regulate antigen-specific Treg function warrants consideration when addressing autoimmune and allergic disease manifestations.

Nevertheless, efficient antigen delivery to LSECs remains challenging. The use of lipid nanoparticles (LNPs) to induce immune tolerance is an active research area, including surface decoration with peptides/major histocompatibility complex (MHC) molecules to serve as alternative antigen-presenting platforms for immune tolerance without co-stimulation. Recent studies indicate that by incorporating mannose ligands onto LNP surfaces, these particles can specifically target LSECs, thereby enhancing immunotolerance through improved cellular uptake, surface epitope presentation, and Treg induction. However, this design relies on a single ligand, potentially leading to imprecise targeting and uptake by hepatocytes or other non-parenchymal cells.

Therefore, there is an urgent need for a targeted LNP system capable of efficiently delivering antigens to LSECs.

### BRIEF SUMMARY OF THE INVENTION

The present invention discloses a lipid nanoparticle (LNP) and its application, comprising cationic ionisable lipids, auxiliary lipids, ligand-modified PEGylated lipids, and cholesterol. The auxiliary lipids and ligand-modified PEGylated lipids target receptors on the surface of liver sinusoidal endothelial cells (LSECs). The LNPs enhance LSEC-specific uptake through multivalent targeting design, encapsulating RNA therapeutics for treating allergic or autoimmune diseases. These RNA therapeutics encode at least one epitope of an antigen implicated in the aetiology of the respective allergic or autoimmune disorder. The LNPs efficiently target LSECs to induce immune tolerance, providing a novel antigen-specific immunomodulatory strategy for treating allergic or autoimmune diseases.

On the one hand, the present invention provides a lipid nanoparticle comprising cationic ionisable lipids, auxiliary lipids, ligand-modified PEGylated lipids, and cholesterol; wherein the ligand in the ligand-modified PEGylated lipid is capable of targeting receptors on the surface of liver sinusoidal endothelial cells, and wherein the auxiliary lipid is selected from lipids capable of targeting receptors on the surface of liver sinusoidal endothelial cells or non-targeting lipids.

The existing technology presents two major challenges: Firstly, the poor targeting capability of lipid nanoparticles, which predominantly rely on non-specific delivery or single-ligand targeting. This results in low uptake efficiency by LSECs, with a disproportionately high uptake rate by non-target cells (such as hepatocytes).Secondly, component design remains ambiguous, with unclear synergistic targeting logic between auxiliary lipids and ligands (e.g., single mannose ligands targeting CD206 exhibit imprecise targeting). The lipid nanoparticles provided by the present invention can be selected as auxiliary lipids that target LSECs surface receptors or auxiliary lipids without targeting function, and can be combined with at least one ligand-modified PEGylated lipid that targets LSECs surface receptors to achieve monovalent or multivalent targeting. When is combined with at least two types of ligand-modified PEGylated lipids the prepared LNPs the auxiliary lipid possesses targeting functionality, or the auxiliary lipid lacks targeting capability but, can multi-valent achieve targeting of receptors on the surface of LSECs, rather than pan-hepatic targeting. This resolves the issue in existing technologies where LNPs accumulate in the liver but exhibit low efficiency in targeting LSECs.

Furthermore, the cationic ionisable lipid is SM102; the auxiliary lipids comprise any one of PS, DOPS, and DPPS; and the ligand-modified PEGylated lipids include at least DSPE-PEG2K-mannose, DSPE-PEG2K-GlcNAc, DSPE-PEG2K-trimannose, DSPE-PEG2K-GalNAc-4-sulphate, DSPE-PEG2K-L-fucose, and DSPE-PEG2K-hyaluronic acid.

The PS (phosphatidylserine) targets SR-E1/LOX-1(Lectin-like Oxidized Low-Density Lipoprotein Receptor-1 (LOX-1, SR-E1) is a type II transmembrane scavenger receptor encoded by the *OLR1* gene, belonging to the Class E scavenger receptor (SR-E) subfamily and C-type lectin superfamily. It mediates binding, internalization, and degradation of oxidized low-density lipoprotein (oxLDL) and diverse ligands (AGEs, apoptotic cells, pathogens, HSPs), and is linked to vascular inflammation,) and SR-H1/STABILIN-1 (SR-H1/Stabilin-1 (also designated FEEL-1, CLEVER-1, MS-1 antigen; gene symbol STAB1) is a large type-I transmembrane glycoprotein and the founding member of the Class H scavenger receptor (SR-H) subfamily. It is a multi-domain scavenger receptor containing fasciclin-like domains, EGF-like repeats, laminin-type EGF-like domains, and a hyaluronan-binding Link module. Primarily expressed on liver, spleen, and lymph node sinusoidal endothelial cells (LSECs/SSECs/LNSECs) and alternatively activated macrophages, it mediates high-capacity endocytosis, recycling, and degradation of diverse ligands including acetylated LDL (acLDL), oxidized LDL (oxLDL), advanced glycation end products (AGEs), hyaluronan, Gram-positive/Gram-negative bacteria, apoptotic cells, and SPARC. It also regulates cell adhesion, lymphocyte homing, angiogenesis, and immunosuppressive signaling, and rapidly cycles between the plasma membrane and early endosomes to maintain tissue homeostasis.) the surface of LSECs.

The DOPS (1,2-dioleoyl-sn-glycero-3-phosphate-L-serine) targets the SR-E1/LOX-1 and SR-H1/STABILIN-1 receptors on LSECs.

The DPPS (1,2-dipalmitoyl-sn-glycero-3-phosphate-L-serine) targets the SR-E1/LOX-1 receptor on LSECs.

The GalNAc-4-sulfate targets the SR-E3/mannose receptor (CD206) on the surface of LSECs; The mannose, trimannose, L-fucose, and GlcNAc target the SR-E3/mannose receptor (CD206) and LSECtin/CLEC4G on the surface of LSECs; The hyaluronic acid targets LYVE-1 on the surface of LSECs. GlcNAc is N-Acetylgalactosamine.

Preferably, the auxiliary lipid is PS.

In certain embodiments, the types of auxiliary lipids exhibiting targeted properties were screened, with PS-based auxiliary lipids yielding the most effective liver-targeting LNPs.

Furthermore, the ligand-modified PEGylated lipids comprised DSPE-PEG2K-mannose, DSPE-PEG2K-GlcNAc, DSPE-PEG2K-trimannose, DSPE-PEG2K-GalNAc-4-sulphate, DSPE-PEG2K-L-fucose DSPE-PEG2K-hyaluronic, and acid.

Preferably, the ligand-modified PEGylated lipids comprise DSPE-PEG2K-trimannose and DSPE-PEG2K-GlcNAc.

In some embodiments, the type and quantity of the ligand-modified PEGylated lipids are selected such that the prepared LNPs exhibit optimal liver targeting efficacy when the combination comprises DSPE-PEG2K-trimannose and DSPE-PEG2K-GlcNAc.

Furthermore, the molar ratios of the cationically ionisable lipid, auxiliary lipid, ligand-modified PEGylated lipid, and cholesterol were 20-70:1-15:1-5:25-45, with the sum of the molar ratios of each component equalling 100%; the N/P ratio of the cationically ionisable lipid was 2-6.

Preferably, when the cationically ionisable lipid is SM102, the N/P ratio of said SM102 is 6.

Furthermore, the particle size of the lipid nanoparticle is 80-200 nm; the lipid nanoparticle encapsulates RNA therapeutics for treating allergic or autoimmune diseases.

The pore diameters within LSECs typically range between 50 and 200 nanometres; consequently, the size of the lipid nanoparticles provided by the present invention can be maintained within the sinusoidal space, thereby facilitating interaction between LSECs and the lipid nanoparticles and enhancing the opportunity for LSECs to internalise the lipid nanoparticles.

Furthermore, the mass ratio of the RNA drug to the lipid nanoparticle is 1 to 10:20 to 100.

Preferably, the mass ratio of the RNA drug to the lipid nanoparticle is 1:40.

Moreover, the RNA is mRNA, comprising a 5'-cap structure, 5'-UTR, coding region, 3'-UTR, and poly A tail.

The mRNA is unstable and carries a negative charge, while the cell membrane surface also carries a negative charge. Electrostatic repulsion makes it difficult for mRNA molecules to cross the cell membrane and enter the cell, thus requiring encapsulation by the lipid nanoparticle to achieve mRNA delivery and intracellular expression.

The 5'-UTR refers to the non-coding region of mRNA located immediately upstream (i.e., 5') of the start codon (the first codon of the mRNA transcript transcribed by the ribosome). The 3'-UTR refers to the non-coding region of mRNA located immediately downstream (i.e., 3') of the stop codon (the codon in the mRNA transcript that signals the termination of translation).The polyA tail is a sequence appended to the 3' terminus of most eukaryotic mRNAs, aiding in the regulation of mRNA stability, transport, and translation. Both the 5'UTR and 3'UTR are typically transcribed from genomic DNA and constitute elements of pre-mature mRNA. Characteristic structural features of mature mRNA (the 5'-cap structure and polyA tail) are usually added to the transcribed mRNA during processing.

Furthermore, the sequences of the 5'UTR and 3'UTR are independently derived from at least one of natural proteins and synthetic proteins.

Preferably, the natural protein comprises any one of α-globulin, β-globulin, and heat shock protein HSP70.

Furthermore, the 5'-UTR comprises a Kozak sequence.

In the present invention, the Kozak sequence is a nucleotide sequence located downstream of the 5'-cap structure in mRNA, which can bind to the promoter to mediate the initiation of translation of mRNA containing the 5'-cap structure.

Furthermore, the coding region comprises at least one epitope encoding an antigen that induces therapeutic allergic or autoimmune diseases.

The epitope is a specific chemical group within the antigen molecule that determines antigen specificity, also known as an antigenic determinant. It serves as the fundamental unit for the specific binding of TCR/BCR and antibodies. Epitopes can be categorised into continuous epitopes (linear epitopes) and discontinuous epitopes (conformational epitopes). In immune responses, T-cell epitopes and B-cell epitopes are distinguished based on the TCR and BCR recognised by the antigen epitope. Epitopes typically comprise no more than 20 amino acids, are recognisable by the body, and can stimulate antibody production. They form the basis of protein antigenicity and constitute the fundamental structure inducing immune responses. Naturally occurring immune reactions cannot recognize all epitopes but concentrate on a relatively small number.

The described epitopes constitute preferred epitopes, selected through AI-assisted design to identify high-affinity, hydrophilic epitopes from antigens that induce therapeutic responses against allergic or autoimmune diseases.

Furthermore, when multiple epitopes are present, these are connected in series via linkers.

The linker connects multiple antigenic epitopes in series to facilitate peptide cleavage upon intracellular release of the epitopes. Moreover, the linker is a flexible linker. Preferably, the linker is a glycine-serine (Gly-Ser) linker or a GPGPG linker.

Furthermore, a targeting sequence is inserted upstream of the coding region.

The targeting sequence enables the antigenic epitope to enter the MHC-II endosomal compartment for peptide presentation to Treg precursor cells. In the present invention, the targeting sequence is preferably the 1-80 amino acid fragment of the invariant chain (Ii), abbreviated as Ii(1-80).whose protein subdomain (an MHC-II molecular chaperone facilitating peptide loading for antigen presentation) enables peptide epitopes to transition from the cytoplasm into MHC-II for presentation to CD4⁺ T cells. The targeting sequence may alternatively comprise the transferrin receptor.

The invention further optimises the codons of the template cDNA transcribed into mRNA to achieve optimal gene expression in non-human cells relative to human cells, utilising their respective tRNA pools. This was accomplished via GenScript's online codon optimisation tool. Additionally, during transcription, uridine is substituted with N1-methylpseudouridine; post-transcription, a 5' cap (either CleanCap or ARCA) and a poly A tail (preferably 100-120 nucleotides in length) are added to the mRNA.

Furthermore, the present invention provides a method for preparing said mRNA, comprising the following steps:
(1) Designing and synthesising cDNA comprising a 5'-UTR, coding region, and 3'-UTR;
(2) Transcribing the cDNA from step (1) into mRNA;
(3) Adding a 5'-cap structure and a poly A tail to the mRNA transcribed in step (2).

Owing to the fragility of RNA therapeutics, employing multiple covalent linkage steps may compromise the stability of both LNPs and mRNA. Therefore, the present invention employs a "one-pot" synthesis method for LNPs and their encapsulated RNA therapeutics, enabling the conjugation of multiple ligands onto the same LNP to ensure specific targeting of LSECs.

In certain embodiments, microfluidic methods are employed for LNP preparation.

The LNPs of the present invention precisely bind to multiple receptors on the surface of LSECs through an innovative multivalent targeting design, efficiently delivering RNA therapeutics encoding disease-associated antigen epitopes. The core mechanism of action is to induce antigen-specific immune tolerance, thereby regulating abnormal immune responses at their root. This mechanism determines that its therapeutic scope is not confined to allergic diseases such as birch pollen allergy and mugwort allergy, nor to autoimmune diseases like type 1 diabetes, myasthenia gravis, toxic diffuse goitre, systemic lupus erythematosus, primary biliary cholecystitis, multiple sclerosis, and Sjögren's syndrome. Rather, it encompasses all allergic and autoimmune diseases sharing the core pathological feature of "disrupted immune tolerance." For allergic diseases, regardless of whether the allergen is pollen, dust mites, food proteins, or pet dander, they fundamentally involve an excessive IgE-mediated immune response to harmless exogenous substances. This invention can be achieved by loading immunodominant epitope RNA corresponding to the allergen, thereby inducing the proliferation of regulatory T cells (Tregs) and suppressing Th2 cell activation to block the allergic reaction cascade. For autoimmune diseases, regardless of whether the pathogenic target is pancreatic β-cells, myelin sheaths, thyroid receptors, or cholangiocytes, the core mechanism involves the abnormal activation of autoreactive T/B cells attacking self-tissues. This invention can specifically induce immune tolerance by loading optimised epitope RNA corresponding to the autoantigen, thereby suppressing autoantibody production and pro-inflammatory factor release to alleviate tissue damage. Provided the disease exhibits the core characteristics of "a clearly identifiable pathogenic antigen and disrupted immune tolerance", targeted therapy can be achieved by screening immunodominant epitopes for the corresponding antigen, optimising the coding sequence of the RNA drug, and utilising the LNP delivery system of the present invention. This approach offers broad applicability and scalability.

Preferably, the allergic diseases include birch pollen allergy and mugwort allergy, while the autoimmune diseases encompass type 1 diabetes mellitus ( ), myasthenia gravis, toxic diffuse goitre, systemic lupus erythematosus, primary biliary cholangitis, multiple sclerosis, and Sjögren's syndrome ( ).

The RNA encapsulated within the lipid nanoparticles for treating the aforementioned birch pollen allergy has a coding region encoding at least one of the amino acid sequences shown in SEQ ID Nos. 2 to 7; the RNA encapsulated within the lipid nanoparticles for treating the aforementioned mugwort allergy has a coding region encoding at least one of the amino acid sequences shown in SEQ ID Nos. 185 to 186;The RNA encapsulated within the lipid nanoparticles for treating the aforementioned Type 1 diabetes has a coding region that encodes at least one amino acid sequence as shown in SEQ ID Nos. 22 to 41; The RNA encapsulated within the lipid nanoparticles for treating the aforementioned myasthenia gravis has a coding region that encodes at least one amino acid sequence as shown in SEQ ID Nos. 64 to 69 or 77 to 79;RNA encapsulated within a lipid nanoparticle for treating toxic diffuse goitre, wherein the coding region encodes at least one amino acid sequence as shown in SEQ ID Nos. 86 to 93; RNA encapsulated within a lipid nanoparticle for treating systemic lupus erythematosus, wherein the coding region encodes at least one amino acid sequence as shown in SEQ ID Nos. 103 to 112;RNA encapsulated within lipid nanoparticles for treating said primary cholangitis, wherein the coding region encodes at least one amino acid sequence as shown in SEQ ID No. 123-131;The RNA encapsulated within the lipid nanoparticle for treating the aforementioned multiple sclerosis has a coding region encoding at least one amino acid sequence as shown in SEQ ID No. 143 to 150; The RNA encapsulated within the lipid nanoparticle for treating the aforementioned Sjögren's syndrome has a coding region encoding at least one amino acid sequence as shown in SEQ ID No. 164 to 173.

Furthermore, the reverse-translated cDNA sequences corresponding to the amino acid sequences shown in SEQ ID Nos. 2-7 are as shown in SEQ ID Nos. 8-13.

In some embodiments, the present invention provides a preferred mRNA for treating birch pollen allergy, wherein the coding region of said mRNA encodes all preferred epitope amino acid sequences as shown in SEQ ID Nos. 2-7, and the reverse-translated cDNA sequence corresponding to the amino acid sequence of its coding region is as shown in SEQ ID No. 16, with a GC content of 56.74%.

Furthermore, the reverse-translated cDNA sequence corresponding to the amino acid sequence shown in SEQ ID Nos. 185-186 is as shown in SEQ ID Nos. 187-188.

In some embodiments, the present invention provides a preferred mRNA for treating Artemisia annua allergy, wherein the coding region of said mRNA encodes all preferred epitope amino acid sequences as shown in SEQ ID Nos. 185-186, and the reverse-translated cDNA sequence corresponding to the amino acid sequence of its coding region is as shown in SEQ ID No. 21.

Furthermore, the reverse-translated cDNA sequence corresponding to the amino acid sequence shown in SEQ ID Nos. 22-41 is as shown in SEQ ID Nos. 42-61.

In some embodiments, the present invention provides a preferred mRNA for treating type 1 diabetes (T1D), wherein the coding region of said mRNA encodes all preferred epitope amino acid sequences as shown in SEQ ID Nos. 22-41, and the reverse-translated cDNA sequence corresponding to the amino acid sequence of its coding region is as shown in SEQ ID No. 62, with a GC content of 55.88%.

Furthermore, the reverse-translated cDNA sequences corresponding to the amino acid sequences shown in SEQ ID Nos. 64-69 and 77-79 are shown in SEQ ID Nos. 70-75 and 80-82.

In certain embodiments, the present invention provides a preferred mRNA for myasthenia gravis (MG), wherein the coding region of said mRNA encodes all preferred epitope amino acid sequences as shown in SEQ ID Nos. 64-69 and 77-79, and the reverse-translated cDNA sequence corresponding to the amino acid sequence of its coding region is as shown in SEQ ID No. 84, with a GC content of 55.08%.

Furthermore, the reverse-translated cDNA sequence corresponding to the amino acid sequence shown in SEQ ID Nos. 86-93 is as shown in SEQ ID Nos. 94-101.

I n some embodiments, the present invention provides a preferred mRNA for toxic diffuse goitre (GD disease), wherein the coding region of said mRNA encodes all preferred epitope amino acid sequences as shown in SEQ ID Nos. 86-93, and the reverse-translated cDNA sequence corresponding to the coding region amino acid sequence is as shown in SEQ ID No. 102, with a GC content of 58.63%.

Furthermore, the reverse-translated cDNA sequence corresponding to the amino acid sequence shown in SEQ ID Nos. 103-112 is shown in SEQ ID Nos. 113-122.

In some embodiments, the present invention provides a preferred mRNA for systemic lupus erythematosus (SLE), wherein the coding region of said mRNA encodes all preferred epitope amino acid sequences as shown in SEQ ID No. 103-112, and the reverse-translated cDNA sequence corresponding to the coding region amino acid sequence is as shown in SEQ ID No. 124, with a GC content of 57.48%.

Furthermore, the reverse-translated cDNA sequence corresponding to the amino acid sequence shown in SEQ ID Nos. 126-131 is as shown in SEQ ID Nos. 132-137.

In some embodiments, the present invention provides an optimised mRNA for primary biliary cholangitis (PBC), wherein the coding region of said mRNA encodes all optimised epitope amino acid sequences as shown in SEQ ID Nos. 126-131, and the reverse-translated cDNA sequence corresponding to the coding region amino acid sequence is as shown in SEQ ID No. 139, with a GC content of 59.87%.

Furthermore, the reverse-translated cDNA sequence corresponding to the amino acid sequence shown in SEQ ID No. 143-150 is as shown in SEQ ID No. 151-158.

In some embodiments, the present invention provides a preferred mRNA for multiple sclerosis (MS), wherein the coding region of said mRNA encodes all preferred epitope amino acid sequences as shown in SEQ ID No. 143-150, and the reverse-translated cDNA sequence corresponding to the amino acid sequence of its coding region is as shown in SEQ ID No. 159, with a GC content of 60.37%.

Furthermore, the reverse-translated cDNA sequence corresponding to the amino acid sequence shown in SEQ ID No. 164-173 is as shown in SEQ ID No. 174-183.

In certain embodiments, the present invention provides a preferred mRNA for Sjögren's syndrome (SS), wherein the coding region of said mRNA encodes all preferred epitope amino acid sequences as shown in SEQ ID No. 164-173, and the reverse-translated cDNA sequence corresponding to the amino acid sequence of its coding region is as shown in SEQ ID No. 184, with a GC content of 57.03%.

Alternatively, the present invention provides the use of the aforementioned lipid nanoparticles in formulations designed to enhance targeting capability towards hepatic sinusoidal endothelial cells.

In some embodiments, the multivalent targeted LNPs provided by the present invention may enhance the ability to target hepatic sinusoidal endothelial cells compared to monovalent targeted LNPs and untargeted LNPs.

Furthermore, the present invention provides the use of the aforementioned lipid nanoparticles in the preparation of formulations for enhancing the efficacy of treating allergic or autoimmune diseases.

In some embodiments, the LNPs provided by this invention treat hay fever caused by birch pollen in allergic diseases by intravenously injecting monovalent, bivalent, trivalent, tetravalent, and pentavalent targeted LNPs carrying mRNAs that treat hay fever (the mRNAs encode six preferred epitopes of the Bet v1 protein, the major allergen in birch pollen) into a mouse model of hay fever. Compared to untreated hay fever mouse models, treated mice exhibited increased proportions of Foxp3⁺ regulatory T cells in lymph nodes and mucosal tissues, alongside reduced levels of IL-4, IL-5, and IL-13.This indicates that monovalent or polyvalent targeted LNPs can, on the one hand, induce the generation of Foxp3⁺ regulatory T cells (Tregs). These regulatory T cells migrate to draining lymph nodes and mucosal tissues, thereby inducing tolerance to allergens; whilst simultaneously suppressing Th2 cells, reducing IgE production, lowering IL-4, IL-5, and IL-13 levels, and limiting degranulation of mast cells and eosinophils, thereby effectively controlling allergic responses. Notably, mice injected with trivalent targeted LNPs (comprising PS+DSPE-PEG2K-trimannose+DSPE-PEG2K-GlcNAc) exhibited the highest proportion of Foxp3⁺ regulatory T cells alongside the lowest levels of IL-4, IL-5, and IL-13, demonstrating optimal therapeutic efficacy. It is thus evident that the LNPs provided by the present invention, loaded with allergen peptide epitopes, can prevent or treat allergic diseases.

In certain embodiments, the LNPs provided by the present invention treat type 1 diabetes mellitus (T1D) in autoimmune diseases by delivering monovalent, bivalent, trivalent, tetravalent, and pentavalent targeted LNPs into an accelerated T1D NOD mouse model. Compared to the accelerated T1D NOD mouse model without LNP injection, the treated mice exhibited a reduction in weight loss and blood glucose levels decreased in all treated mice. However, only mice injected with the trivalent targeted LNPs (composition: PS+DSPE-PEG2K-trimannose+DSPE-PEG2K-GlcNAc) exhibited blood glucose levels approaching those of normal mice, alongside the lowest pancreatic tissue damage scores. It is thus evident that the LNPs provided by the present invention, loaded with peptide epitopes of self-antigens, can prevent or treat autoimmune diseases.

In certain embodiments, the LNPs provided by the present invention are also employed for treating artemisia allergy, myasthenia gravis, toxic diffuse goitre, systemic lupus erythematosus, primary cholangitis, multiple sclerosis, and Sjögren's syndrome. Wherein the LNPs respectively encapsulate mRNA encoding the antigen that induces various allergic or autoimmune diseases, and mRNA encoding all preferred epitopes of . The therapeutic efficacy of injecting trivalent targeted LNPs (components comprising PS+DSPE-PEG2K-trimannose+DSPE-PEG2K-GlcNAc) into mouse models of each disease significantly surpasses that of monovalent or other multivalent targeted LNPs.

### BENEFICIAL EFFECTS OF THE INVENTION

1. The LNPs provided by this invention exhibit high targeting precision and uptake efficiency: the innovative multivalent targeting design (targeting auxiliary lipids + at least one ligand-modified PEGylated lipid) precisely binds to multiple receptors on the surface of LSECs, resulting in a higher proportion of liver-specific uptake;
2. The invention identifies an optimised composition formulation for LNPs, further enhancing targeting specificity and therapeutic efficacy against allergic and autoimmune diseases;
3. The invention optimises mRNA drugs encapsulated within LNPs, wherein the mRNA coding region comprises at least one preferred antigenic epitope;
4. This invention identifies preferred epitopes of antigens inducing various allergic or autoimmune diseases, providing the corresponding cDNA sequences for reverse translation at . It also offers therapeutic solutions for birch pollen allergy, Artemisia annua allergy, type 1 diabetes, myasthenia gravis, toxic diffuse goitre, systemic lupus erythematosus, primary cholangitis, multiple sclerosis, and Sjögren's syndrome;
5. The LNPs provided by the present invention may be developed as vaccines and immunomodulators for the prevention or treatment of allergic diseases and autoimmune diseases, exhibiting strong antigen specificity and low side effects.

### BRIEF DESCRIPTION OF THE DRAWINGS

Figure 1 shows Example 2, the distribution fluorescence as imaged by of mice injected with LNPs prepared using different cationic ionisable lipids in an in vivo imaging system (IVIS);
Figure 2A-2C shows obtained from in vivo imaging system (IVIS) imaging of mice injected with LNPs prepared using different cationic ionisable lipids in Example 2 the fluorescence intensity results;
Figure 3 shows in Example 2the distribution of different fluorescent signals in various organs of mice injected with LNPs prepared using different cationic ionisable lipids ;
Figure 4A-4C: 2 Different fluorescence proportions in various organs and the intensity of different fluorescence in the liver of mice injected with LNPs prepared from different cationic ionisable lipids in Example2;
Figure 5 shows the fluorescence distribution in various organs of mice injected with SM102-LNPs (multivalent targeting), MC3-LNPs (multivalent targeting), SM102-LNPs (monovalent targeting 1), SM102-LNPs (monovalent targeting 2), and SM102-LNPs (monovalent targeting 3) in mice in Example2;
Figure 6 shows the fluorescence intensity in the livers of mice administered SM102-LNPs (multivalent targeting), MC3-LNPs (multivalent targeting), SM102-LNPs (monovalent targeting 1), SM102-LNPs (monovalent targeting 2), and SM102-LNPs (monovalent targeting 3) in mice in Example 2;
Figure 7 shows the schematic diagram of the complete mRNA structure in Example 6;
Figure 8 shows the experimental protocol for constructing accelerated T1D NOD mouse models in Example 7 (red arrows) and the treatment protocol for accelerated T1D NOD mouse models along with monitoring protocols (blue and green arrows);
Figure 9 illustrates the distribution of different epitopes within the α-subunit sequence of AChR in Example 9;
Figure 10 presents the proliferation responses to different epitopes within the α-subunit of AChR from four distinct MG patients (Pt 3/7/10/11) in Example 9;
Figure 11: Frequency of recognition of different α-subunit peptides from AChR in Example 9 by seven responsive MG patients (indicated by dotted light grey bars);
Figure 12 shows the binding of the RO60 epitope to HLA alleles in Example 14;
Figure 13: TRIM21 epitope binding to HLA alleles in Example 14;
Figure 14: Binding patterns of the lupus La epitope with HLA alleles in Example 14;
Figure 15 illustrates the binding configuration of the SPTN1 epitope with HLA alleles in Example 14.

### DETIAL EMBODIMENTS

The present invention shall now be described in further detail with reference to the accompanying drawings and embodiments. It should be noted that the embodiments described below are intended to facilitate understanding of the invention and are not intended to limit the scope thereof.

### Example 1: Preparation of Lipid Nanoparticles

The lipid nanoparticles (LNPs) prepared in this example comprise the following components: SM102, phosphatidylserine (PS), DSPE-PEG2K-trimannose, DSPE-PEG2K-GlcNAc, cholesterol, and the encapsulated RNA drug. Herein, SM102 is a cationic ionisable lipid, phosphatidylserine is an auxiliary lipid, and DSPE-PEG2K-trimannose and DSPE-PEG2K-GlcNAc are ligand-modified PEGylated lipids; The RNA drug is an RNA therapeutic for treating allergic or autoimmune diseases, specifically mRNA comprising a 5'-cap structure, 5'-UTR, coding region, 3'-UTR, and poly A tail, wherein the coding region encodes at least one epitope of an antigen causing allergic or autoimmune diseases.

The specific preparation method is as follows: Components for preparing LNPs are prepared using a microfluidic method, with an ethanol phase to aqueous phase ratio of 1:3 (the ethanol phase contains the lipid components, the aqueous phase contains the RNA drug), a total flow rate of 12 mL/min, and a molar mass ratio of lipid components SM102:PS:Chol:DSPE-PEG2K-trimannose:DSPE-PEG2K-GlcNAc = 50:10:38.5:1:0.5. The N/P ratio of SM102 is 6, and the mass ratio of RNA drug to lipid nanoparticles is 1-10:20-100 (preferably 1:40).The prepared LNPs were dialysed using PBS.

### Example 2: Screening of Cationic Ionisable Lipids and Their N/P Ratios in Lipid Nanoparticles

The LNPs in this embodiment were prepared using the method described in Example 1, with the cationic ionisable lipid SM102 replaced by MC3. When SM102 was used as the cationic ionisable lipid, the N/P ratios were set to 2, 3, 4, and 6 respectively; when MC3 was used, the N/P ratio was set to 4. To facilitate subsequent characterisation, experiments, and in vivo tracking of the LNPs, all prepared LNPs were loaded with two mRNAs encoding enhanced green fluorescent protein (EGFP) and luciferase (Luc), respectively. DiR was incorporated into the LNP formulation for labelling (DiR molar ratio: 0.3; to maintain a total molar sum of 100%, the molar ratio of Chol was adjusted from 38.5 to 38.2).

First, the physicochemical properties of the prepared LNPs were characterized, including encapsulation efficiency (EE), particle size (Size), polydispersity index (PDI), zeta potential, and acid dissociation constant (pKa). The characterization results are presented in Table 1 below.

**Table 1. Characterization results of physicochemical properties for LNPs prepared with different cationically ionisable lipids**

| Cationic ionisable lipid and its N/P ratio | EE (%) | Size (nm) | PDI | Zeta potential (mV) | pKa |
|---|---|---|---|---|---|
| SM102-N/P=2 | 96.1 | 117.6 | 0.04 | -10.32 | 6.517 |
| SM102-N/P=3 | 96.3 | 112.5 | 0.05 | -10.69 | 6.465 |
| SM102-N/P=4 | 96.8 | 121.3 | 0.06 | -11.15 | 6.537 |
| SM102-N/P=6 | 97.6 | 109.2 | 0.03 | -9.52 | 6.239 |
| MC3-N/P=4 | 95.4 | 102.7 | 0.05 | -12.04 | 6.331 |

As shown in Table 1, when the cationic ionisable lipid was SM102 with N/P ratios ranging from 2 to 6, the encapsulation efficiency of the resulting LNPs consistently exceeded that of LNPs prepared using MC3 with an N/P ratio of 4.Therefore, SM102 is the preferred cationic ionisable lipid. Comparing the physicochemical characterisation results of LNPs prepared from SM102 with different N/P ratios, those with an N/P ratio of 6 exhibited the highest encapsulation rate, smallest particle size, and most uniform particle size distribution. Considering these factors comprehensively, an N/P ratio of 6 is optimally selected.

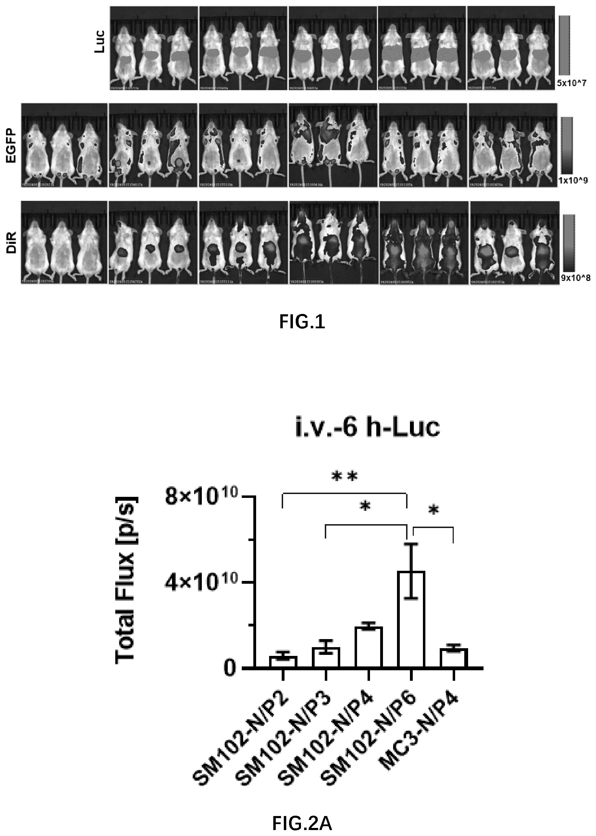

Furthermore, LNPs prepared with different cationic ionisable lipids were injected. An equal volume of PBS served as the control subcutaneously into the mouse tail at 20 µg mRNA/mouse. Six hours later, fluorescence imaging was performed using an in vivo imaging system (IVIS) to observe the location of LNPs and mRNA expression. Results are shown in Figures 1-2.,the qualitative and quantitative fluorescence intensity results , it was found the cationic ionisable lipid in LNPs Due to the strong background signal from EGFP, it holds no comparative value. However by comparing of DiR and Luc that when was SM102 the N/P ratio of SM102 was 6 both DiR and, the fluorescence signals of Luc were stronger and concentrated in the liver region of the mice.

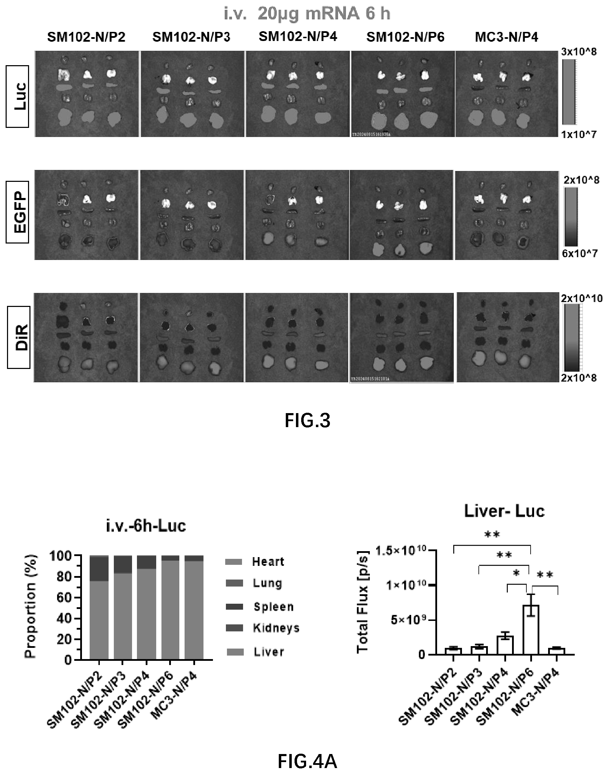

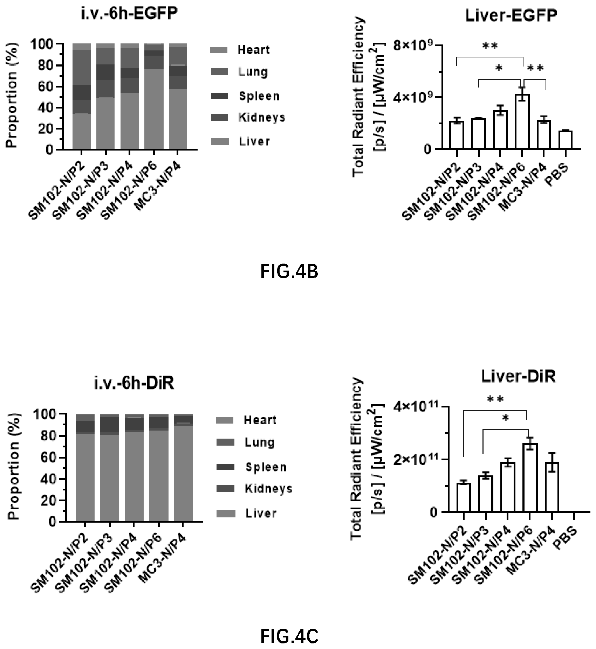

Furthermore, mice from each group were euthanised, and their organs were harvested for imaging. The luminescence or fluorescence intensity of these organs-including the kidneys, spleen, lungs, liver, heart, and lymph nodes-was compared. Results are presented in Figures 3-4. Comparison of DiR fluorescence proportion across organs and DiR fluorescence intensity in the liver revealed minimal variation in DiR proportion within the liver across groups However, .mice injected with SM102-N/P6 LNPs exhibited the highest DiR fluorescence intensity in the liver. Comparing the fluorescence proportion of Luc and EGFP each organ and the fluorescence intensity of Luc and EGFP in the liver across groups revealed in mice injected with exhibited the highest fluorescence proportion of Luc and EGFP in the liver that both alongside the highest fluorescence intensity in the liver SM102-N/P6 LNPs ,.These results indicate that when SM102 serves as the cationic ionisable lipid in LNPs and its N/P ratio is 6, is. the capacity for specific liver targeting and mRNA translation maximised

In, SM102 is the preferred cationic ionisable lipid for LNPs, with an optimal N/P ratio of 6 for said SM102. summary

### Example 3: In Vivo Targeting Validation of LNPs

This embodiment selected the two LNPs prepared in Example 2 when the cationic ionisable lipids were SM102-N/P=6 and MC3-N/P=4 respectively as experimental subjects, designated as SM102-LNPs (multivalent targeting) and MC3-LNPs (multivalent targeting).Concurrently, the combination of DSPE-PEG2K-trimannose and DSPE-PEG2K-GlcNAc within the SM102-LNPs (multivalent targeting) was replaced with a single DSPE-PEG2K, designated as SM102-LNPs (monovalent targeting 1).. Furthermore, the phosphatidylserine in the SM102-LNPs (multivalent targeting) was replaced with the common auxiliary lipid DSPC, while the combination of DSPE-PEG2K-trimannose and DSPE-PEG2K-GlcNAc was substituted with a single DSPE-PEG2K-trimannose, designated as SM102-LNPs(monovalent targeting 2); replacing the phosphatidylserine in the SM102-LNPs (multivalent targeting) with the common auxiliary lipid DSPC, and replacing the combination of DSPE-PEG2K-trimannose and DSPE-PEG2K-GlcNAc with a single DSPE-PEG2K-GlcNAc, denoted as SM102-LNPs(Monovalent Targeting 3).

Twenty micrograms of mRNA per LNP formulation were injected subcutaneously into the mouse tail. Six hours later, fluorescence imaging was performed using an in vivo imaging system (IVIS) to observe LNP localisation and mRNA expression. Subsequently, mice from each group were euthanised, and organs including kidney, spleen, lung, liver, heart, and lymph nodes were harvested for imaging. The luminescence or fluorescence intensity of these organs was compared.

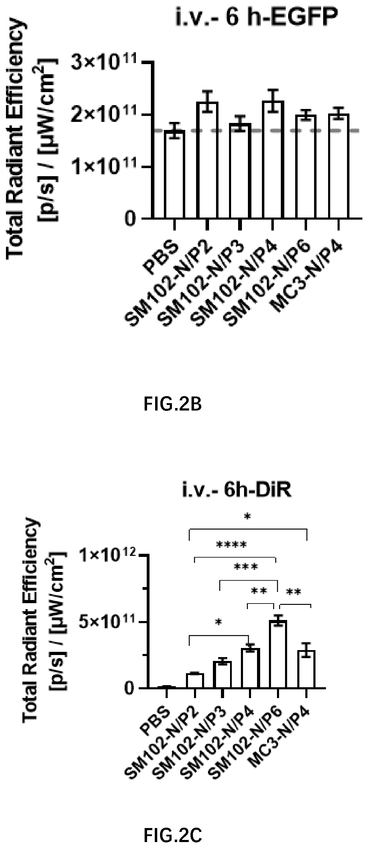

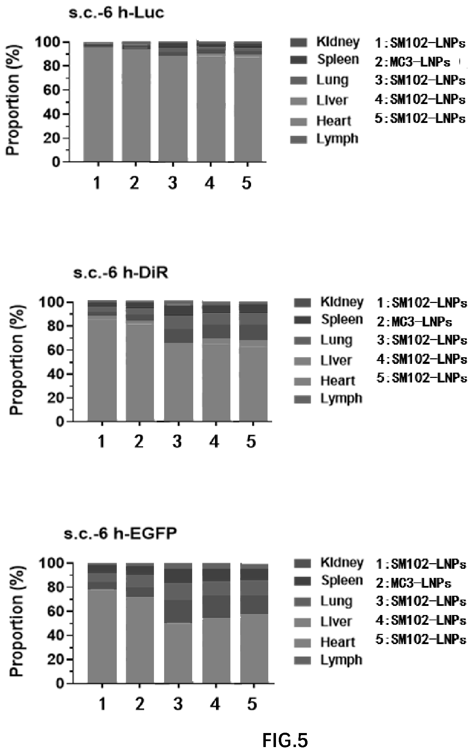

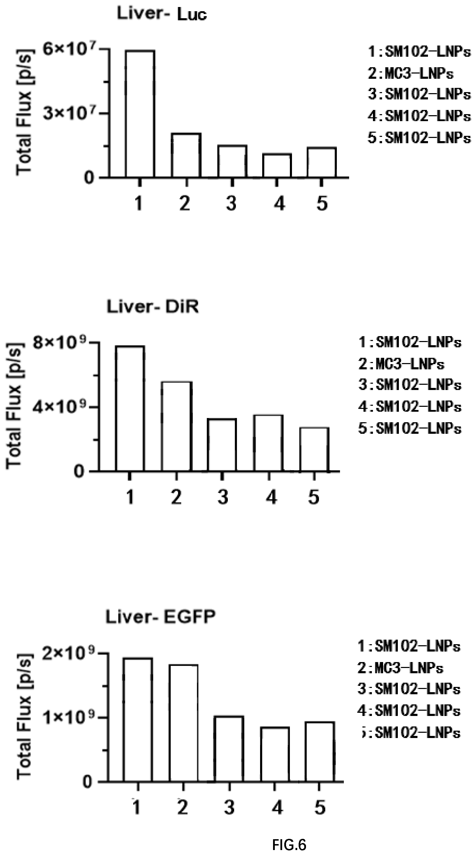

The fluorescence proportion of DiR, Luc, and EGFP in various organs across five groups of mice, along with the fluorescence intensity of DiR, Luc, and EGFP in the liver, are shown in Figures 5 to 6. Comparing the DiR fluorescence proportion in Figure 1 with the DiR fluorescence intensity in the liver in Figure 2, the DiR fluorescence proportion in the liver and the DiR fluorescence intensity in the liver of mice injected with SM102-LNPs (multivalent targeting) and MC3-LNPs (multivalent targeting) were significantly higher than those in mice injected with the other three monovalent targeting LNPs; Comparing the Luc and EGFP fluorescence proportions in Figure 1 with the Luc and EGFP fluorescence intensities in the liver in Figure 2,The proportion of Luc and EGFP fluorescence in the liver, as well as the intensity of Luc and EGFP fluorescence in the liver, were significantly higher in mice injected with SM102-LNPs (multivalent targeting) and MC3-LNPs (multivalent targeting) compared to mice injected with the other three monovalent targeting LNPs. These results indicate that multivalent targeted LNPs exhibit superior specific targeting to the liver and mRNA translation capabilities compared to monovalent targeted LNPs. Furthermore, SM102-LNPs (multivalent targeting) demonstrated superior targeting efficacy to SM102-LNPs (monovalent targeting).

### Example 4: Effect of Auxiliary Lipids on Lipid Nanoparticle Targeting

The PS, DSPE-PEG2K-trimannose, and DSPE-PEG2K-GlcNAc components in the LNPs prepared in Example 1 all possess the ability to target LSEC surface receptors, with PS serving as the auxiliary lipid. This example replaces the type of auxiliary lipid, with replaceable auxiliary lipids including DOPS (1,2-dioleoyl-sn-glycero-3-phospho-L-serine), DPPS (1,2-dipalmitoyl-sn-glycero-3-phospho-L-serine), and DSPC(distearoylphosphatidylcholine). Both DOPS and DPPS possess the ability to target surface receptors on LSECs, with DOPS targeting the SR-E1/LOX-1 and SR-H1/STABILIN-1 receptors on LSECs, and DPPS targeting the SR-E1/LOX-1 receptor on LSECs.

Prepare four types of LNPs using PS and its interchangeable auxiliary lipids according to the method of Example 1. Each LNP encapsulates two mRNAs encoding EGFP and Luc respectively, with DiR incorporated into the LNP composition for labelling (DiR molar ratio: 0.3; to maintain total molar sum at 100%, the molar ratio of Chol was adjusted from 38.5 to 38.2).

Five micrograms of each of the five LNP formulations were administered subcutaneously into the mouse tail. Six hours post-injection, mice from each group were euthanised, and organs were harvested for imaging to compare luminescence or fluorescence intensity. Organs included kidneys, spleen, lungs, liver, heart, and lymph nodes. Results demonstrated that the proportion of fluorescence in the liver of each group of mice was significantly higher than in other organs. The proportions of DiR, Luc, and EGFP fluorescence in the livers of mice from each group are shown in Table 2.

**Table 2. Proportion of DiR, Luc, and EGFP fluorescence in the liver of mice across groups**

| Organ and Fluorescence Type | | Percentage of DiR, Luc, and EGFP fluorescence in mouse livers following injection of LNPs containing different co-lipids (%) | | | |
|---|---|---|---|---|---|
| | | PS | DOPS | DPPS | DSPC |
| | DiR | 83.4 | 76.8 | 72.9 | 66.7 |
| Liver | Luc | 97.8 | 90.7 | 92.3 | 85.2 |
| | EGFP | 69.4 | 62.9 | 60.7 | 54.2 |

According to the data in Table 2, when the auxiliary lipids were the targeted PS, DOPS, and DPPS, the prepared LNPs exhibited higher fluorescence proportions of DiR, Luc, and EGFP in the liver compared to LNPs prepared with the untargeted DSPC. This indicates that the incorporation of targeted auxiliary lipids enhances the liver-specific targeting efficacy of LNPs. concurrently, LNPs prepared with PS exhibited the highest fluorescence ratios for DiR, Luc, and EGFP, indicating superior liver targeting efficacy and the strongest capacity for mRNA translation within the liver. Consequently, PS is the preferred targeted auxiliary lipid.

### Example 5: Effect of Ligand-Modified PEGylated Lipids on the Targeting Properties of Lipid Nanoparticles

Building upon Example 4, this example employs PS as the preferred auxiliary lipid while varying the quantity and type of ligand-modified PEGylated lipids. The ligand-modified PEGylated lipids included, in addition to DSPE-PEG2K-trimannose and DSPE-PEG2K-GlcNAc, DSPE-PEG2K-mannose, DSPE-PEG2K-GalNAc-4-sulphate,DSPE-PEG2K-L-fucose, and DSPE-PEG2K-hyaluronic acid.

Combinations of PEGylated lipids with different ligand modifications were selected to form LNPs, as shown in Table 3 below.

**Table 3. Combinations of PEGylated lipids with different ligand modifications**

| LNP ID | DSPE-PEG2K-Tri-Gal | DSPE-PEG2K-GlcNAc | DSPE-PEG2K-mannose | DSPE-PEG2K-GalNAc-4-sulphate | DSPE-PEG2K-L-fucose | DSPE-PEG2K-Hyaluronic Acid |
|---|---|---|---|---|---|---|
| 1 | √ | | | | | |
| 2 | | √ | | | | |
| 3 | | | √ | | | |
| 4 | | | | √ | | |
| 5 | | | | | | |
| 6 | | | | | | √ |
| 7 | √ | √ | | | | |
| 8 | √ | | √ | | | |
| 9 | √ | | | √ | | |
| 10 | √ | | | | √ | |
| 11 | √ | | | | | √ |
| 12 | | √ | √ | | | |
| 13 | | √ | | √ | | |
| 14 | | √ | | | √ | |
| 15 | | √ | | | | √ |
| 16 | | | √ | √ | | |
| 17 | | | √ | | √ | |
| 18 | | | √ | | | √ |
| 19 | | | | √ | √ | |
| 20 | | | | √ | | √ |
| 21 | | | | | √ | √ |
| 22 | √ | √ | √ | | | |
| 23 | √ | √ | | √ | | |
| 24 | √ | √ | | | √ | |
| 25 | √ | √ | | | | √ |
| 26 | √ | √ | √ | √ | | |
| 27 | √ | √ | √ | | √ | |
| 28 | √ | √ | √ | | | √ |
| 29 | √ | √ | | √ | √ | |
| 30 | √ | √ | | √ | | √ |
| 31 | √ | √ | | | √ | √ |

In Table 3 above, LNPs 1-6 comprise PEGylated lipids modified with one type of ligand (with a molar mass ratio of 1.5 for the single lipid), while LNPs 7-21 comprise PEGylated lipids modified with two types of ligands ( , with a molar mass ratio of (0.75):(0.75) for the two lipids ).LNPs 22-25 comprised three ligand-modified PEGylated lipids ( , with a molar ratio of (0.5):(0.5):(0.5)), while LNPs 25-31 comprised four ligand-modified PEGylated lipids ( , with a molar ratio of (0.375):(0.375):(0.375):(0.375)). : (0.375) : (0.375)) . All LNPs encapsulated two mRNAs encoding EGFP and Luc respectively, with DiR incorporated for labelling (DiR molar ratio 0.3; to maintain total molar sum at 100%, Chol molar ratio was adjusted from 38.5 to 38.2).

Each of the 31 LNP formulations was administered at 5 µg per mouse via subcutaneous tail injection. Six hours post-injection, mice from each group were euthanised, and organs including kidney, spleen, lung, liver, heart, and lymph nodes were harvested for imaging. The luminescence or fluorescence intensity of these organs was compared. Results demonstrated that the proportion of fluorescence in the liver of each group of mice was significantly higher than in other organs. The proportions of DiR, Luc, and EGFP fluorescence in the livers of mice across all groups are shown in Table 4.

**Table 4. Proportion of DiR, Luc, and EGFP fluorescence in the liver of mice across groups**

| LNP ID | Percentage of DiR, Luc and EGFP fluorescence in mouse liver following injection of LNPs containing PEGylated lipid combinations modified with different ligands | | |
|---|---|---|---|
| | DiR | Luc | EGFP |
| 1 | 68.5 | 88.2 | 50.1 |
| 2 | 63.6 | 83.7 | 46.3 |
| 3 | 59.2 | 85.4 | 45.6 |
| 4 | 53.8 | 82.6 | 42.5 |
| 5 | 62.4 | 84.5 | 40.9 |
| 6 | 55.5 | 87.2 | 42.3 |
| 7 | 83.4 | 97.8 | 69.4 |
| 8 | 78.2 | 92.5 | 53.1 |
| 9 | 75.7 | 89.3 | 59.8 |
| 10 | 74.3 | 94.6 | 62.5 |
| 11 | 72.1 | 95.4 | 58.2 |
| 12 | 76.9 | 90.7 | 51.3 |
| 13 | 67.8 | 96.8 | 66.7 |
| 14 | 72.6 | 94.9 | 54.3 |
| 15 | 73.4 | 92.1 | 58.1 |
| 16 | 76.8 | 93.5 | 53.6 |
| 17 | 80.9 | 91.7 | 61.8 |
| 18 | 69.2 | 91.3 | 59.5 |
| 19 | 68.7 | 94.6 | 58.9 |
| 20 | 79.3 | 96.4 | 57.2 |
| 21 | 65.9 | 94.2 | 55.8 |
| 22 | 53.5 | 82.8 | 40.1 |
| 23 | 52.1 | 86.5 | 32.7 |
| 24 | 51.3 | 89.2 | 41.5 |
| 25 | 59.8 | 82.7 | 38.2 |
| 26 | 38.5 | 77.3 | 25.1 |
| 27 | 37.2 | 75.9 | 27.6 |
| 28 | 36.1 | 74.5 | 22.7 |
| 29 | 35.4 | 78.2 | 28.9 |
| 30 | 34.2 | 71.8 | 23.9 |
| 31 | 33.1 | 73.5 | 26.3 |

Based on the data in Table 4, comparing the fluorescence ratios of DiR, Luc, and EGFP in the livers of mice injected with LNPs 1-6, 7-21, 22-25, and 26-31 LNPs, it was found that LNPs 1-6,22-25, and 26-31 LNPs exhibited inferior liver targeting efficacy and mRNA translation capacity compared to LNPs 7-21.indicating that LNPs prepared with one, three, or four types of ligand-modified PEGylated lipids demonstrated inferior liver targeting efficacy and mRNA translation capacity in the liver compared to LNPs prepared with two types of ligand-modified PEGylated lipids. Comparing experimental data from LNPs 7-21, mice injected with LNP 1 exhibited higher fluorescence ratios of DiR, Luc, and EGFP in the liver than those injected with any other LNPs 8-21. This demonstrates that LNP 1 exhibits the strongest liver targeting efficacy and the highest capacity for mRNA translation within the liver. These results demonstrate that targeting efficacy does not increase with the number of ligand-modified PEGylated lipids; rather, a combination of two such lipids yields superior targeting. The optimal choice is DSPE-PEG2K-trimannose + DSPE-PEG2K-GlcNAc, as excessive ligands reduce targeting efficiency due to steric hindrance and receptor competition.

In summary, when PS serves as the auxiliary lipid, the preferred ligand-modified PEGylated lipids are DSPE-PEG2K-trimannose and DSPE-PEG2K-GlcNAc.

### Example 6: Treatment of White Birch Pollen Allergy

This example demonstrates that LNPs prepared in Example 1 can be employed for treating birch pollen-induced hay fever.

### Prediction and design of antigenic epitope peptides, and preparation of mRNA

Bet v 1 protein is a major birch pollen allergen identified in pollen from Betula pendula and Betula pendula var. pendula. Its amino acid sequence, as shown in SEQ ID NO.1, induces IgE binding in over 95% of birch pollen allergy sufferers. IgE triggers Type I hypersensitivity reactions, manifesting as allergic rhinitis, allergic conjunctivitis, hay fever, allergic asthma, bee venom allergy, and food allergy.

Using the Immunological Epitope Database (IEDB) and the NetMHCIIpan 4.1 EL tool, a search was conducted for MHC class II epitopes . Table 5 below lists epitopes within the Bet v1 protein exhibiting high binding percentile values with the aforementioned MHC class II epitopes.

**Table 5. Epitopes in the Bet v1 protein with high binding percentile values for the described MHC-II class epitopes**

| Sequence ID | Peptide Amino Acid Sequence | Start | End | Peptide amino acid count | Allele | Peptide amino acid count | Median binding percentile |
|---|---|---|---|---|---|---|---|
| 1 | KAFILDGDNLFPKVA | 21 | 35 | 15 | HLA-DRB3*01:01 | 5 | 0.08 |
| 2 | DHTNFKYNYSVIEGG | 76 | 90 | 15 | HLA-DRB3*02:02 | 16 | 0.13 |
| 3 | KYNYSVIEGGPIGDT | 81 | 95 | 15 | 5 HLA-DRB1*01:01 | 17 | 0.15 |
| 4 | EGFPFKYVKDRVDEV | 61 | 75 | 15 | HLA-DRB1*04:05 | 13 | 0.16 |
| 5 | KISNKYHTKGDHEVK | 116 | 130 | 15 | HLA- DQA1*04:01/DQB1*04:02 | 24 | 0.16 |
| 6 | KYNYSVIEGGPIGDT | 81 | 95 | 15 | HLA-DRB1*07:01 | 17 | 0.21 |
| 7 | FPKVAPQAISSVENI | 61 | 75 | 15 | HLA- DQA1*04:01/DQB1*04:02 | 13 | 0.32 |
| 8 | GETLLRAVESYLLAH | 36 | 50 | 15 | HLA-DRB1*04:01 | 8 | 0.4 |
| 9 | GETLLRAVESYLLAH | 61 | 75 | 15 | HLA- DQA1*01:01/DQB1*05:01 | 13 | 0.41 |
| 10 | EGFPFKYVKDRVDEV | 81 | 95 | 15 | HLA-DRB1*09:01 | 17 | 0.59 |
| 11 | NEIKIVATPDGGSIL | 31 | 45 | 15 | HLA-DRB4*01:01 | 7 | 0.68 |
| 12 | GETLLRAVESYLLAH | 141 | 155 | 15 | HLA-DRB1*07:01 | 29 | 0.72 |
| 13 | GETLLRAVESYLLAH | 141 | 155 | 15 | HLA-DRB1*15:01 | 29 | 0.72 |
| 14 | EGFPFKYVKDRVDEV | 61 | 75 | 15 | HLA-DRB3*01:01 | 13 | 0.81 |
| 15 | NEIKIVATPDGGSIL | 101 | 115 | 15 | HLA-DRB1*04:01 | 21 | 0.96 |
| 16 | DGDNLFPKVAPQAIS | 26 | 40 | 15 | HLA-DRB1*08:02 | 6 | 1.1 |

Six Bet v 1 preferred epitope peptides were selected from Table 5 above based on HLA allele coverage, as shown in Table 6 below.

**Table 6. Six Bet v 1 preferred epitope peptides**

| Corresponding Sequence Number in Table 5 | Epitope Sequence | HLA Alleles (Count) | Associated HLA Alleles | Optimal Median Binding Percentile |
|---|---|---|---|---|
| 10 | EGFPFKYVKDRVDEV | 4 | DRB104:05, DQA104:01/DQB104:02, | 0.16 |
| 6 | KYNYSVIEGGPIGDT | 3 | DRB101:01, DRB107:01, DRB1*09:01 | 0.15 |
| 12 | GETLLRAVESYLLAH | 2 | *DRB107:01,* DRB115:01 | 0.72 |
| 1 | KAFILDGDNLFPKVA | 1 | DRB3*01:01 | 0.08 |
| 2 | DHTNFKYNYSVIEGG | 1 | DRB3*02:02 | 0.13 |
| 5 | KISNKYHTKGDHEVK | 1 | *DQA104:*01/DQB104:02 | 0.16 |

The six table-position peptides in Table 6 were flanked with sequences and reverse-translated into cDNA for mRNA design, as shown in Table 7 below.

**Table 7. Back-translated sequences of preferred epitope peptides**

| Corresponding sequence number in Table 5 | SE Q ID NO. | Epitope with flanking sequences | Position | Reverse-translated cDNA sequence | SEQ ID NO. |
|---|---|---|---|---|---|
| 10 | 2 | FPEGFPFKYVKD RVDEVDH | 61-75 | TTTCCGGAAGGCTTTCCGTTTAAATATGTGAAAGATCG CGTGGATGAAGTGGATCAT | 8 |
| 6 | 3 | NFKYNYSVIEGG PIGDTLE | 81-95 | AACTTTAAATATAACTATAGCGTGATTGAAGGCGGCCC GATTGGCGATACCCTGGAA | 9 |
| 12 | 4 | EMGETLLRAVES YLLAHSD | 141-155 | GAAATGGGCGAAACCCTGCTGCGCGCGGTGGAAAGCT ATCTGCTGGCGCATAGCGAT | 10 |
| 1 | 5 | LFKAFILDGDNLF PKVAPQ | 21-35 | CTGTTTAAAGCGTTTATTCTGGATGGCGATAACCTGTT TCCGAAAGTGGCGCCGCAG | 11 |
| 2 | 6 | EVDHTNFKYNYS VIEGGPI | 76-90 | GAAGTGGATCATACCAACTTTAAATATAACTATAGCGT GATTGAAGGCGGCCCGATT | 12 |
| 5 | 7 | ILKISNKYHTKGD HEVKAE | 116-130 | ATTCTGAAAATTAGCAACAAATATCATACCAAAGGCG ATCATGAAGTGAAAGCGGAA | 13 |

Furthermore, a complete cDNA sequence was constructed, wherein the cDNA comprises a 5'UTR (containing the Kozak sequence), a coding region, and a 3'UTR.

The cDNA sequence of the 5'UTR is:
GAAATAAGAGAGAAAAGAAGAGTAAGAAGAAATATAAGAGCCACC.

The cDNA sequence of the 3'UTR is:

CTCTTCCTCTATGCTCTTCCTGTGCTCTTCCTCTATGCTCTTCCTCTCAAAAAAA
AAAAAAAGCATAAATAACTAAAATACCCAGTCAAGTTACTATTAGTAGATAG.

The coding region encodes the targeting sequence and at least one epitope from Table 7; in this embodiment, six epitopes were selected. The six cDNA sequences from Table 13 were connected via a flexible linker (in this embodiment, the GPGPG linker was chosen, whose reverse translation into cDNA sequence is GGCCCGGGCCCGGGC) as part of the coding region, to enable peptide cleavage upon epitope release within the cell. The targeting sequence enables the antigenic epitope to enter the MHC-II endosomal compartment for peptide presentation to Treg precursor cells. In this embodiment, the targeting sequence comprises the 1-80 amino acid fragment of the invariant chain (Ii), as shown in SEQ ID NO. 14, abbreviated as Ii(1-80), with its reverse-translated cDNA sequence shown in SEQ ID NO. 15.The protein subdomain of this fragment (an MHC-II chaperone protein facilitating peptide loading into MHC-II for antigen presentation) enables peptide epitopes to transition from the cytoplasm into MHC-II for presentation to CD4⁺ T cells. The target sequence may alternatively be the transferrin receptor.

Furthermore, the coding region cDNA underwent codon optimisation to achieve optimal gene expression in non-human cells relative to human cells, utilising the GenScript online codon optimisation tool. The optimised nucleotide sequence of the coding region is shown in SEQ ID NO.16, exhibiting a GC content of 56.74%, with the sequence encoding Ii(1-80) positioned upstream of the sequences encoding the six epitopes.

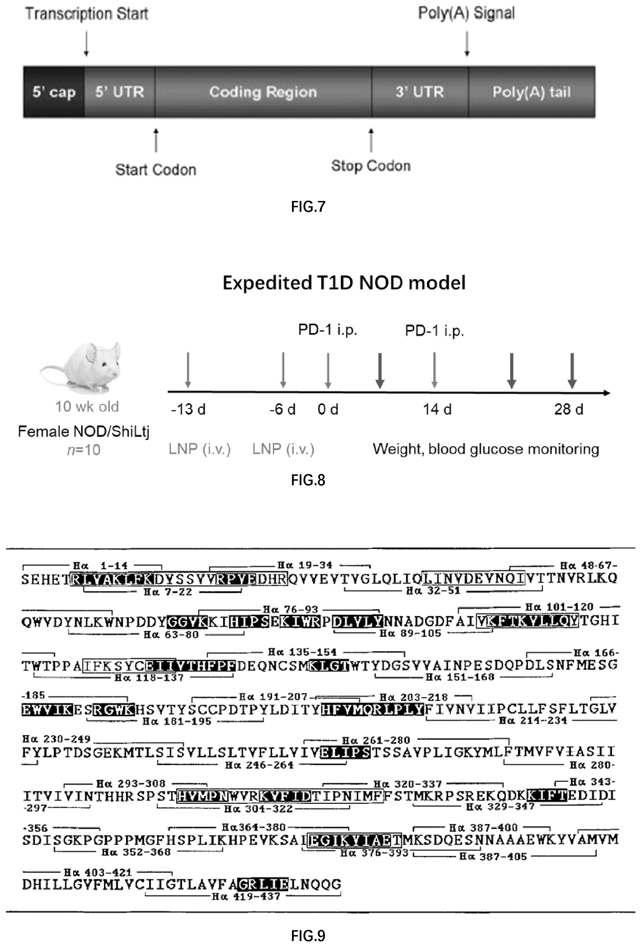

Subsequently, the optimised cDNA was transcribed into mRNA. During transcription, uridine was substituted with N1-methylpseudouridine. Post-transcription, a 5' cap (either CleanCap or ARCA) and a poly A tail (100-120 nucleotides in length) were added to the mRNA. The complete mRNA sequence is shown in Figure 7.

Preferably, mRNA transcription may be achieved by inserting the coding region cDNA into the pTNT plasmid.

### 2. Preparation of LNPs

The LNPs prepared using DSPC as the auxiliary lipid in Table 2 of Example 3, along with LNPs 1 and 7 (identical to Example 1), 22 and 26 from Table 4 of Example 5,22 and 26 from Table 4 of Example 5 were used to encapsulate the mRNA prepared in this example. These were designated as monovalent targeted LNPs, bivalent targeted LNPs, trivalent targeted LNPs, tetravalent targeted LNPs, and pentavalent targeted LNPs respectively.

### 3. LNPs for treating birch pollen-induced hay fever

Construction of hay fever model mice: An animal model of pollen allergy was established using the subcutaneous (S.C.) sensitisation immunisation method with 25 µg of silver birch pollen extract. The challenge step involved inhalation of 1% Bet v1 pure protein.

LNPs prepared with DSPC as the auxiliary lipid (as per Table 2 in Example 3) and LNPs 1, 7 (same as Example 1), 22, and 26 (as per Table 4 in Example 5) were used.22, and 26 from Table 4 of Example 5, respectively, were loaded with mRNA prepared in this example, designated as monovalent targeted LNPs, bivalent targeted LNPs, trivalent targeted LNPs, tetravalent targeted LNPs, and pentavalent targeted LNPs.

The bivalent, trivalent, quadrivalent, and pentavalent targeted LNPs were administered intravenously at 5 µg per mouse to hay fever model mice. The control group comprised model mice injected with an equivalent volume of blank LNPs. Following a treatment period, the proportion of Foxp3⁺ regulatory T cells and expression levels of inflammatory cytokines IL-4, IL-5, and IL-13 were assessed in lymph nodes and mucosal tissues across all groups. Results are presented in Table 8 below.

**Table 8: Proportion of Foxp3⁺ Regulatory T Cells and Expression Levels of Inflammatory Cytokines IL-4, IL-5, and IL-13**

| Test item | Monovalent Targeted LNPs | Bivalent-targeted LNPs | Trivalent Targeted LNPs (as in Example 1) | Quadrivalent Targeted LNPs | Pentavalent Targeted LNPs | Control |
|---|---|---|---|---|---|---|
| Foxp3⁺ regulatory T cell proportion (%) | 8.5 | 13.8 | 18.3 | 11.2 | 9.7 | 4.2 |
| IL-4 (pg/mL) | 18.7 | 12.3 | 7.8 | 14.5 | 16.2 | 26.5 |
| IL-5 (pg/mL) | 23.5 | 15.7 | 9.2 | 18.4 | 20.1 | 32.8 |
| IL-13 (pg/mL) | 21.3 | 14.1 | 8.5 | 16.8 | 18.6 | 29.4 |

LNP therapy induces the generation of Foxp3⁺ regulatory T cells (Tregs), which migrate to draining lymph nodes and mucosal tissues, thereby eliciting tolerance responses to allergens; while simultaneously suppressing Th2 cells, reducing IgE production, lowering IL-4, IL-5, and IL-13 levels, and limiting degranulation of mast cells and eosinophils, thereby effectively controlling allergic responses. Table 9 results demonstrate that in all groups of mice injected with targeted LNPs, the proportion of Foxp3⁺ Tregs significantly increased compared to controls, while IL-4, IL-5, and IL-13 levels significantly decreased. Comparing mice injected with the various targeted LNPs, the trivalent targeted LNPs (as in Example 1) yielded the highest proportion of Foxp3⁺ Tregs and the lowest levels of IL-4, IL-5, and IL-13, indicating the most effective treatment for allergic reactions.

These results demonstrate that the LNPs encapsulating allergen peptide epitopes can prevent or treat allergic diseases, particularly those induced by birch pollen or birch extracts, such as hay fever or oral allergy syndrome, thus exhibiting considerable application potential.

### Example 7: Treatment of Artemisia allergen allergy

This example verifies that LNPs prepared in Example 1 can be used to treat mugwort allergy.
1. Prediction and Design of Antigenic Episites and Preparation of mRNA
   , a major birch allergen identified in pollen from Betula pendula and Betula pendula var. pendula, induces IgE binding in over 95% of birch pollen allergy sufferers. Immunoglobulin E (IgE) triggers Type I hypersensitivity reactions, manifesting as allergic rhinitis, allergic conjunctivitis, hay fever, allergic asthma, bee venom allergy, and food allergies.

The full-length protein coding sequence for mugwort pollen protein Art v1 is shown as SEQ ID NO. 17.

This example utilised the NetMHCIIpan 4.1 eluate (EL) predictor from the IEDB to forecast epitope binding to MHC-II in Art v1.Whilst binding affinity assesses a peptide's capacity to bind MHC molecules, eluate (EL) prediction further integrates the likelihood of that peptide being naturally processed and presented. This enhances the prediction's propensity to identify genuine T-cell epitopes. Table 9 below presents the ranked epitope prediction results:

**Table 9. Art v1 epitope binding to MHC-II**

| Allele | Start | End | Peptide | Score | Sequence |
|---|---|---|---|---|---|
| H2-IAb | 63 | 77 | APPGAAPPPAAGGSP | 0.322 | 3.4 |
| H2-IAb | 64 | 78 | PPGAAPPPAAGGSPS | 0.2974 | 3.7 |
| H2-IAb | 56 | 70 | SPPGATPAPPGAAPP | 0.2226 | 5 |
| H2-IAb | 62 | 76 | PAPPGAAPPPAAGGS | 0.2178 | 5.1 |
| H2-IAb | 57 | 71 | PPGATPAPPGAAPPP | 0.2025 | 5.4 |
| H2-IAb | 59 | 73 | GATPAPPGAAPPPAA | 0.1847 | 6 |
| H2-IAb | 58 | 72 | PGATPAPPGAAPPPA | 0.1707 | 6.4 |
| H2-IAb | 55 | 69 | KSPPGATPAPPGAAP | 0.1492 | 7.1 |
| H2-IAb | 65 | 79 | PGAAPPPAAGGSPSP | 0.1415 | 7.4 |
| H2-IAb | 61 | 75 | TPAPPGAAPPPAAGG | 0.1299 | 8 |
| H2-IAb | 60 | 74 | ATPAPPGAAPPPAAG | 0.0911 | 11 |
| H2-IAb | 52 | 66 | DCSKSPPGATPAPPG | 0.1008 | 11 |
| H2-IAb | 51 | 65 | FDCSKSPPGATPAPP | 0.0831 | 12 |
| H2-IAb | 54 | 68 | SKSPPGATPAPPGAA | 0.075 | 13 |
| H2-IAb | 69 | 83 | PPPAAGGSPSPPADG | 0.0766 | 13 |
| H2-IAb | 70 | 84 | PPAAGGSPSPPADGG | 0.0564 | 16 |
| H2-IAb | 68 | 82 | APPPAAGGSPSPPAD | 0.057 | 16 |
| H2-IAb | 50 | 64 | YFDCSKSPPGATPAP | 0.0475 | 18 |
| H2-IAb | 48 | 62 | FCYFDCSKSPPGATP | 0.0439 | 19 |
| H2-IAb | 79 | 93 | PPADGGSPPPPADGG | 0.0409 | 20 |
| H2-IAb | 53 | 67 | CSKSPPGATPAPPGA | 0.0412 | 20 |

Subsequently, based on the data in Table 9 above and considering hydrophilicity, hydrophobicity, and water solubility, APPGAAPPPAAGGSP was selected as the preferred epitope 1 (SEQ ID NO: 185) and SCFCYFDCSKSPPGATP as the preferred epitope 2 (SEQ ID NO: 186).The reverse-translated cDNA sequence corresponding to the preferred epitope 1 is shown in SEQ ID NO: 187, while the reverse-translated cDNA sequence corresponding to the preferred epitope 2 is shown in SEQ ID NO: 188.

Furthermore, a complete cDNA sequence is constructed, wherein the cDNA comprises a 5'UTR (including the Kozak sequence), a coding region, and a 3'UTR. The nucleotide sequences of the 5'UTR and 3'UTR are identical to those in Example 6. The coding region encodes the target sequence and at least one of the aforementioned key epitope/peptide segment sequences. The cDNA sequence of the epitope/peptide segment is connected to the targeting sequence via a linker. The leader sequence and linker sequence are as described in Example 6.In this embodiment, four coding regions cDNA sequences are provided based on all the aforementioned epitopes/peptide segments. The coding region cDNA undergoes further codon optimisation using the method described in Example 6. The optimised coding region cDNA sequences are shown respectively as SEQ ID NO.18 to 21, with GC contents of 63.53%, 57.49%, 60.39% and 57.79% respectively. Among these, SEQ ID NO.18 contains epitope 1, SEQ ID NO.19 contains epitope 2, SEQ ID NO.20 contains epitopes 1 and 2, and SEQ ID NO.21 also incorporates both preferred epitopes.

Furthermore, the optimised cDNA was transcribed into mRNA using the same method as in Example 6.

### 2. Preparation of LNPs

LNPs prepared using DSPC as the auxiliary lipid in Table 2 of Example 3, along with LNPs 1 and 7 (identical to Example 1), 22 and 26 from Table 4 of Example 5, were prepared. These were designated as monovalent targeted LNPs, bivalent LNPs, and LNPs 22 and 26 respectively.22, and 26 from Table 4 of Example 5 were used to encapsulate the mRNA prepared in this example (encoding region cDNA sequence as shown in SEQ ID NO. 21), designated as monovalent targeted LNPs, bivalent targeted LNPs, trivalent targeted LNPs, tetravalent targeted LNPs, and pentavalent targeted LNPs respectively.

### 3. LNPs for the treatment of Artemisia annua allergy

An animal model of pollen allergy was established using subcutaneous (S.C.) injection of Artemisia annua pollen extract for sensitisation. The procedure was as follows: BALB/c mice were subcutaneously injected with 25 µg of Artemisia annua pollen extract on days 1, 8, and 15 for three sensitisation sessions. Subsequently, on days 22, 23, and 24, mice were challenged daily with inhalation of 1% Art v1 purified protein. Blood samples were collected to measure total IgE, antigen-specific IgE, and antigen-specific IgG levels to validate sensitisation efficacy. Subsequently, on days 26, 28, 30, and 32, subcutaneous injections of 2 µg each of the five prepared LNPs were administered. Comparisons were made against normal mice (blank control group) and model mice injected with an equivalent volume of blank LNPs (positive control group).Subsequently, 1% Art v1 pure protein was administered via inhalation daily on days 37, 38, 39, 40, and 41. On day 42, animals were euthanised, and blood samples, bronchoalveolar lavage fluid (BALF) samples, lung tissue, and spleen tissue were collected for further analysis. Paraffin sections were prepared from the collected lung tissue. H&E staining was employed to evaluate inflammatory cell infiltration in the lungs of mice across all groups. Results demonstrated that, compared with the blank control group, the positive control group exhibited marked inflammatory cell infiltration in the bronchioles. Compared with the positive control group, mice treated with the five LNP formulations showed significantly reduced inflammatory cell infiltration and tracheal fibrosis in the bronchioles. Scoring of inflammatory infiltration in the bronchioles of mice across all groups yielded the results presented in Table 10.

**Table 10: Bronchial Inflammatory Infiltration Scores in Mice Across Groups**

| Test Item | Treatment Group | | | | | | |
|---|---|---|---|---|---|---|---|
| | Blank Control | Positive Control | Monovalent Targeted LNPs | Bivalent Targeted LNPs | Trivalent targeted LNPs | Quadrivalent targeted LNPs | Pentavalent targeted LNPs |
| Inflammatory infiltration score | 0.2 | 4.3 | 2.5 | 1.7 | 0.6 | 1.5 | 2.8 |

According to the data in Table 10, compared with the positive control, the bronchial inflammatory infiltration scores decreased in mice treated with all five LNPs. However, only the mice treated with the trivalent targeted LNPs exhibited scores closest to the blank control (normal mice).

The cell counts, IgE levels, IgG levels, IgG1 levels, IgG2a levels, proportion of CD4⁺CD25⁺Foxp3⁺cells (regulatory T cells (Tregs)), and IL-10 content in BALF are shown in Table 11 below.

**Table 11. Cell counts in BALF across groups**

| Test item | Treatment group | | | | | | |
|---|---|---|---|---|---|---|---|
| | Blank control | Positive Control | Monovalent Targeted LNPs | Bivalent Targeted LNPs | Trivalent Targeted LNPs | Quaternary-targeted LNPs | Pentavalent targeted LNPs |
| Cell count (×10⁴) | 12 | 71 | 65 | 48 | 15 | 43 | 52 |
| IgE level | 3.4 | 8.7 | 8.0 | 6.2 | 3.6 | 6.7 | 7.5 |
| IgG level | 136 | 429 | 375 | 244 | 142 | 293 | 362 |
| IgG1 level | 98 | 242 | 217 | 187 | 136 | 164 | 205 |
| IgG2a level | 21 | 23 | 20 | 19 | 22 | 24 | 23 |
| CD4⁺ CD25⁺ Foxp3⁺ Cell Proportion (%) | 50 | 56 | 63 | 67 | 79 | 60 | 53 |
| IL-10 | 13 | 5 | 17 | 25 | 34 | 19 | 16 |

According to the data in Table 11, compared with the positive control group, the five LNP treatment groups exhibited reduced total cell counts, IgE levels, IgG levels, and IgG1 levels in BALF, alongside increased Treg proportion and IL-10 content, while IgG2a levels remained largely unchanged. However, the trivalent targeted LNP group exhibited the lowest levels of total cells, IgE, IgG, and IgG1 in BALF, alongside the highest Treg proportion and IL-10 content, demonstrating the most potent therapeutic effect.

It is evident that subcutaneous injection of LNPs expressing the Art v1 Artemisia pollen protein epitope significantly induces Treg production and IL-10 cytokine secretion, thereby suppressing pollen protein-induced allergic pulmonary inflammation.

### Example 8: Treatment of Type 1 Diabetes

This example demonstrates that LNPs prepared in Example 1 can be employed for the treatment of type 1 diabetes (T1D).

### 1. Prediction and Design of Antigenic Epitope Peptides and Preparation of mRNA

Type 1 diabetes (T1D) arises from the disruption of immune tolerance, whereby autoreactive T cells (particularly CD4⁺ and CD8⁺ T cells) recognise and attack insulin-producing β cells. These cells target multiple islet antigens, including insulin (INS), glutamic acid decarboxylase 65 (GAD65), insulinoma-associated protein 2 (IA-2), ZnT8, and IGRP. Substantial evidence supports the pivotal role of CD4+ T cells in T1D. Their significance is underscored by strong genetic associations with specific HLA class II haplotypes.CD4+ T cells not only drive autoimmune attacks but also promote the production of islet-specific autoantibodies-often generated prior to clinical symptom onset and associated with epitope spreading as β-cell damage progresses. Furthermore, these cells are crucial for generating Tregs that may suppress disease, a mechanism supporting strategies targeting LSECs via tolerogenic nanoparticles to exploit MHC-II-mediated antigen presentation to naive T cells.

This embodiment has selected a total of 20 human epitopes from eight T1D-associated antigens, including insulin, GAD65, chromogranin A, IAPP, IGRP, ZnT8, IA2, and InsB-mimetic epitopes, as well as HIPs (insulin peptide-fused epitopes with other antigen fragments).

The epitope screening in this example focuses on sequences interacting with HLA-DRB1*0401-an allele that pairs with DQA1* 03:01 or DQB1*03:02 to form the "DR4" high-risk haplotype (Noble et al. J Autoimmunity 64, 2015: 101e112).Notably, DRB1*04:01 possesses a unique motif capable of presenting human T1D-associated insulin antigen epitopes (Tait et al. Eur J Immunogenet. 1995; 22(4):289-97).However, T1D risk arises from the synergistic effects of DR/DQ molecules: DRB4*01:01 (DRB4), as a minor HLA-DR product, constitutes part of the high-risk DR4/DQ8 haplotype (James et al. J Immunol 2018; doi:10.4049/jimmunol.1800723).DRB4 also exhibits structural homology with HLA-DR4 alleles associated with other autoimmune diseases such as multiple sclerosis, rheumatoid arthritis, and Lyme arthritis. This embodiment employs DRB1*0401 and its associated DR4 haplotype epitopes to develop a T1D therapeutic strategy involving tolerance-inducing nanoparticles delivering multi-epitope mRNA.

This example utilised the NetMHCipan_el 4.1 server to predict preferred peptide segments (epitopes) capable of binding to DRB1*0401 and its associated DR4 haplotypes among the eight T1D-associated antigens. The prediction results are presented in Table 12 below.

**Table 12. Predicted preferred epitopes for eight T1D-associated antigens**

| Serial No . | Epitope | Position | Amino Acid Sequence and Its Reverse-Translated cDNA Sequence | SEQ ID NO. |
|---|---|---|---|---|
| 1 | Ins B | 9-23 | CGSHLVEALYLVCGERGFF | 22 |
| | | | TGCGGCAGCCATCTGGTGGAAGCGCTGTATCTGGTGTGCGGCGAACGCGGCT TTTTT | 42 |
| 2 | GAD6 5 | 274-300 | RLIAFTSEHSHFSLKKGAAALGIGTDSVILI | 23 |
| | | | CGCCTGATTGCGTTTACCAGCGAACATAGCCATTTTAGCCTGAAAAAGGCGCGG CGGCGCTGGGCATTGGCACCGATAGCGTGATTCTGATT | 43 |
| 3 | Ins B | 9-23 R22E | CGSHLVEALYLVCGEEGFF | 24 |
| | | | TGCGGCAGCCATCTGGTGGAAGCGCTGTATCTGGTGTGCGGCGAAGAAGGCT TTTTT | 44 |
| 4 | Chg A | 341-355 | SKRWSKMDQLAKELTAEKR | 25 |
| | | | AGCAAACGCTGGAGCAAAATGGATCAGCTGGCGAAAGAACTGACCGCGGAA AAACGC | 45 |
| 5 | Hip 2.5 | InsC+ ChgA | SLQPLALWSKMDQL | 26 |
| | | | AGCCTGCAGCCGCTGGCGCTGTGGAGCAAAATGGATCAGCTG | 46 |
| 6 | GA D6 5 | 114-128 | QDVMNILLQYVVKSFDRST | 27 |
| | | | CAGGATGTGATGAACATTCTGCTGCAGTATGTGGTGAAAAGCTTTGATCGCA GCACC | 47 |
| 7 | Hip 10 | InsC+ ChgA | SLQPLALLEGQEEE | 28 |
| | | | AGCCTGCAGCCGCTGGCGCTGCTGGAAGGCCAGGAAGAAGAA | 48 |
| 8 | Hip 11 | Ins+Ins | SLQPLALEAEDLQV | 29 |
| | | | AGCCTGCAGCCGCTGGCGCTGGAAGCGGAAGATCTGCAGGTG | 49 |
| 9 | Hip 12 | Ins+Ins | SLQPLALGIVEQCC | 30 |
| | | | AGCCTGCAGCCGCTGGCGCTGGGCATTGTGGAACAGTGCTGC | 50 |
| 10 | Hip 14 | Ins+I APP | SLQPLALNAVEVLK | 31 |
| | | | AGCCTGCAGCCGCTGGCGCTGAACGCGGTGGAAGTGCTGAAA | 51 |
| 11 | IAPP | 5-13 | ILKLQVFLIVLSV | 32 |
| | | | ATTCTGAAACTGCAGGTGTTTCTGATTGTGCTGAGCGTG | 52 |
| 12 | GAD 65 | 553-572 | K-V-N-F-F-R-M-V-I-S-N-P-A-A-T-H-Q-D-I-D | 33 |
| | | | AAGTGTAACTTTTTCGCATGGTGATTAGCAACCCGGCGGCGACCCATCAGG ATATTGAT | 53 |
| 13 | IGRP | 215-230 | FLFLFAVGFYLLLRVLNIDL | 34 |
| | | | TTTCTGTTTCTGTTTGCGGTGGGCTTTTATCTGCTGCTGCGCGTGCTGAACATT GATCTG | 54 |
| 14 | GAD6 5 | 248-257 | SNMYAMMIARFKMF | 35 |
| | | | AGCAACATGTATGCGATGATGATTGCGCGCTTTAAAATGTTT | 55 |
| 15 | Ins | 73-90 | GPGAGSLQPLALEGSLQKRGIV | 36 |
| | | | GGCCCGGGCGCGGGCAGCCTGCAGCCGCTGGCGCTGGAAGGCAGCCTG CAGAAACGCGGCATTGTG | 56 |
| 16 | Ins | 40-55 | ALYLVCGERGFFYTPKTRRE | 37 |
| | | | GCGCTGTATCTGGTGTGCGGCGAACGCGGCTTTTTTATACCCCGAAAACCC GCCGCGAA | 57 |
| 17 | Zn T8 | 186-194 | CAVAANIVLTVVL | 38 |
| | | | TGCGCGGTGGCGGCGAACATTGTGCTGACCGTGGTGCTG | 58 |
| 18 | IRGP | 265-273 | LGVLFGLGFAINS | 39 |
| | | | CTGGGCGTGCTGTTTGGCCTGGGCTTTGCGATTAACAGC | 59 |
| 19 | GAD6 5 | 553-582 | GDKVNFFRMVISNPAATHQDIDFLIEEIERLGQD | 40 |
| | | | GGCGATAAAGTGAACTTTTTCGCATGGTGATTAGCAACCCGGCGGCGACCC ATCAGGATATTGATTTTCTGATTGAAGAAATTGAACGCCTGGGCCAGGAT | 60 |
| 20 | IA-2 | 618-631 | GPEGAHGDTTFEYQDLCR | 41 |
| | | | GGCCCGGAAGGCGCGCATGGCGATACCACCTTTGAATATCAGGATCTGTGCC GC | 61 |

Furthermore, a complete cDNA sequence is constructed, wherein the cDNA comprises a 5'UTR (including the Kozak sequence), a coding region, and a 3'UTR. The nucleotide sequences of the 5'UTR and 3'UTR are identical to those in Example 6. The coding region encodes the target sequence and at least one epitope sequence from Table 11, with multiple epitopes connected via linkers as part of the coding region. The leader sequence and linker sequence are identical to those in Example 6. In this example, the coding region of the cDNA sequence encompasses all reverse-translated cDNA sequences from Table 11. The coding region cDNA undergoes further codon optimisation using the method described in Example 6. The optimised coding region cDNA sequence is shown in SEQ ID NO: 62, with a GC content of 55.88%.

Furthermore, the optimised cDNA is transcribed into mRNA using the method described in Example 6.

### 2. Preparation of LNPs

LNPs prepared using DSPC as the auxiliary lipid in Table 2 of Example 3, along with those from entries 1 and 7 (identical to Example 1), 22 and 26 in Table 4 of Example 5, were utilised. These LNPs were designated as monovalent targeted LNPs, bivalent targeted LNPs, trivalent targeted LNPs, and tetravalent targeted LNPs, respectively.22, and 26 from Table 4 of Example 5 were loaded with the mRNA prepared in this example, designated as monovalent targeted LNPs, bivalent targeted LNPs, trivalent targeted LNPs, tetravalent targeted LNPs, and pentavalent targeted LNPs respectively.

### 3. LNPs for T1D Therapy

Female NOD. ShiLtJ mice typically develop spontaneous hyperglycaemia between 10 and 14 weeks of age, with an incidence rate of 70-90%.

This study employed an accelerated T1D NOD mouse model, wherein PD-1 blockade disrupted immune tolerance to accelerate the autoimmune diabetes process. The specific protocol involved intraperitoneal injection of anti-PD-1 antibody (5 mg/kg) in 10-week-old female NOD mice, administered in two doses on days 0 and 14 (as indicated by red arrows in Figure 8 ).This model rapidly induces type 1 diabetes (defined as two consecutive blood glucose readings >250 mg/dL). By disrupting regulatory immune pathways, this approach accelerates diabetes onset (Fife et al. J Exp Med 2006; Nishimura et al. 2001), providing a reliable platform for immunomodulatory interventions in T1D research.

To evaluate the efficacy of the aforementioned LNP preparations, monovalent targeted LNPs, bivalent targeted LNPs, trivalent targeted LNPs, tetravalent targeted LNPs, and pentavalent targeted LNPs were dispersed in PBS to form 100 µL solutions (2.7 µg mRNA). These were administered via intravenous injection (injection timing corresponding to the blue arrows in Figure 8 ) in two separate doses to accelerated T1D NOD female mice. The positive control group comprised accelerated T1D NOD female mice injected with an equivalent volume of blank LNPs, while the negative control group comprised normal mice injected with an equivalent volume of blank LNPs.

Body weight and blood glucose levels were monitored in the seven groups of mice. Blood glucose monitoring included glycated haemoglobin (HbA1c) and fasting plasma glucose (FPG). Fasting plasma glucose (FPG) and oral glucose tolerance test (OGTT) are current diagnostic methods for diabetes; however, HbA1c serves as a stable indicator reflecting chronic blood glucose levels, aligning more closely with the definition of diabetes and better reflecting long-term blood glucose levels and the risk of chronic complications.

Body weight changes revealed steady increases in the negative control group (normal mice), sustained decreases in the positive control group (accelerated T1D NOD mice), and overall upward trends in mice injected with monovalent targeted LNPs, divalent targeted LNPs, trivalent targeted LNPs, tetravalent targeted LNPs, and pentavalent targeted LNPs all exhibited an overall upward trend in body weight. Among these, mice injected with trivalent targeted LNPs demonstrated a weight gain pattern most closely resembling that of normal mice. This indicates that accelerated T1D NOD mice exhibited weight loss, a hallmark manifestation of diabetes, while treatment with monovalent or multivalent LNPs mitigated weight decline, with trivalent targeted LNPs demonstrating the most effective weight loss mitigation.

Changes in blood glucose levels from weeks 0 to 8 for each group are presented in Table 13 below.

**Table 13: Changes in blood glucose levels in mice across groups from weeks 0 to 8**

| Mouse treatment | Blood glucose level (mmol/L) | | | | |
|---|---|---|---|---|---|
| | Week 0 | Week 1 | Week 3 | Week 6 | Week 8 |
| Single-dose injection of monovalent targeted LNPs | 7.9 | 10.5 | 22.7 | 31.3 | 32.8 |
| Injection of bivalent targeted LNPs | 7.2 | 8.4 | 17.3 | 19.0 | 18.1 |
| Injection of trivalent targeted LNPs | 6.8 | 7.2 | 9.6 | 10.9 | 10.2 |
| Injection of tetravalent targeted LNPs | 5.7 | 7.3 | 15.8 | 18.5 | 18.2 |
| Injection of pentavalent targeted LNPs | 7.6 | 9.4 | 18.2 | 23.5 | 21.8 |
| Positive control (model mice) | 6.3 | 9.3 | 26.1 | 40.7 | 38.4 |
| Negative control (normal mice) | 5.9 | 6.5 | 7.4 | 7.8 | 6.6 |

Table 13 data indicate that blood glucose levels in negative control mice remained stable within the normal range, whereas positive control mice exhibited a sharp rise in blood glucose that persisted at elevated levels. Mice injected with monovalent or multivalent targeted LNPs effectively reduced blood glucose levels compared to positive control mice over weeks 0-8. However, when comparing the therapeutic effects of monovalent versus multivalent targeted LNPs, only mice injected with trivalent targeted LNPs exhibited blood glucose levels closest to those of negative control mice.

The percentage of HbA1c relative to total haemoglobin in each group after 8 weeks is shown in Table 14 below.

**Table 14: Percentage of HbA1c relative to total haemoglobin in mice across groups after 8 weeks**

| Mouse treatment | Percentage of HbA1c relative to total haemoglobin (%) |
|---|---|
| Monovalent targeted LNPs injection | 8.5 |
| Injection of bivalent targeted LNPs | 6.2 |
| Injection of trivalent targeted LNPs | 4.6 |
| Injection of tetravalent targeted LNPs | 6.9 |
| Injection of pentavalent targeted LNPs | 7.3 |
| Positive control (model mice) | 10.7 |
| Negative control (normal mice) | 4.3 |

Comparing the therapeutic effects in mice treated with monovalent or multivalent targeted LNPs, the proportion of HbA1c decreased in all groups following LNP treatment. However, only mice injected with trivalent targeted LNPs exhibited an HbA1c proportion closest to that of the negative control mice.

The histopathological scores (HE-stained islet damage severity) of pancreatic tissue sections from each group after 8 weeks are presented in Table 15.

**Table 15: Pathological scores of HE-stained pancreatic tissue sections in each group after 8 weeks**

| Mouse treatment | Pathological Score |
|---|---|
| Monovalent targeted LNPs injection | 3.5 |
| Injection of bivalent targeted LNPs | 2.2 |
| Injection of trivalent targeted LNPs | 1.3 |
| Injection of tetravalent targeted LNPs | 2.5 |
| Injection of pentavalent targeted LNPs | 2.9 |
| Positive control (model mice) | 4.2 |
| Negative control (normal mice) | 0.6 |

Table 15 data indicate that pancreatic islets in positive control mice exhibited significantly greater damage compared to negative control mice. However, treatment with either monovalent or multivalent targeted LNPs reduced the extent of islet damage in mice. Notably, only mice treated with trivalent targeted LNPs demonstrated the least islet damage, with scores most closely resembling those of normal mice.

The combined experimental results demonstrate that trivalent targeted LNPs significantly reduce blood glucose levels and maintain long-term glycaemic control near normal ranges when used to treat diabetes, while markedly reducing pancreatic islet damage.

This embodiment successfully designed a multi-epitope mRNA capable of tandemly encoding T1D-associated antigens, along with its associated product (LNPs). This formulation significantly reduces blood glucose and glycated haemoglobin levels in diabetic models while effectively controlling disease progression.

### Example 9: Treatment of Myasthenia Gravis

This example validates that LNPs prepared in Example 1 can be used to treat myasthenia gravis (MG).

### 1. Prediction and Design of Antigenic Epitope Peptides and Preparation of mRNA

Myasthenia gravis (MG) is a chronic autoimmune neuromuscular disorder caused by autoantibody-mediated disruption of neuromuscular junction (NMJ) signalling, resulting in muscle weakness, fatigue, and potentially respiratory failure in severe cases. The hallmark of MG is autoantibodies targeting key NMJ proteins, with primary pathogenic targets being the acetylcholine receptor (AChR), muscle-specific kinase (MuSK), and low-density lipoprotein receptor-related protein 4 (LRP4).Approximately 85% of MG patients are AChR-positive, where CD4⁺ T-cell-dependent autoreactive B-cell activation generates pathogenic anti-AChR autoantibodies. These antibodies induce receptor internalisation, complement-mediated postsynaptic membrane lysis, and impaired neuromuscular transmission, resulting in progressive muscle weakness, ptosis, and dysphagia or dyspnoea. In contrast, MuSK-positive MG (5-10%) involves IgG4 autoantibodies disrupting AChR assembly, while LRP4-positive MG is rarer with an unclear pathophysiology. MG dysimmunity is strongly associated with HLA genetics, particularly HLA-DR3 and HLA-DQ8, which play pivotal roles in antigen presentation and auto ic T/B cell responses.

The primary pathogenic targets in MG are AChR and MuSK; consequently, this embodiment will screen for optimal epitopes on these two pathogenic targets.

### (1) Selection of AChR epitopes

Research findings indicate that the α-subunit of the nicotinic AChR (amino acid sequence as shown in SEQ ID NO.63) is crucial in the pathogenesis of the autoimmune paralytic disease MG. This is because it contains both epitopes that dominate the anti-AChR antibody response and epitopes recognised by CD4+ AChR-specific helper T (Th) cells. The distribution of distinct epitopes within the α-subunit of the described AChR is illustrated in Figure 9.

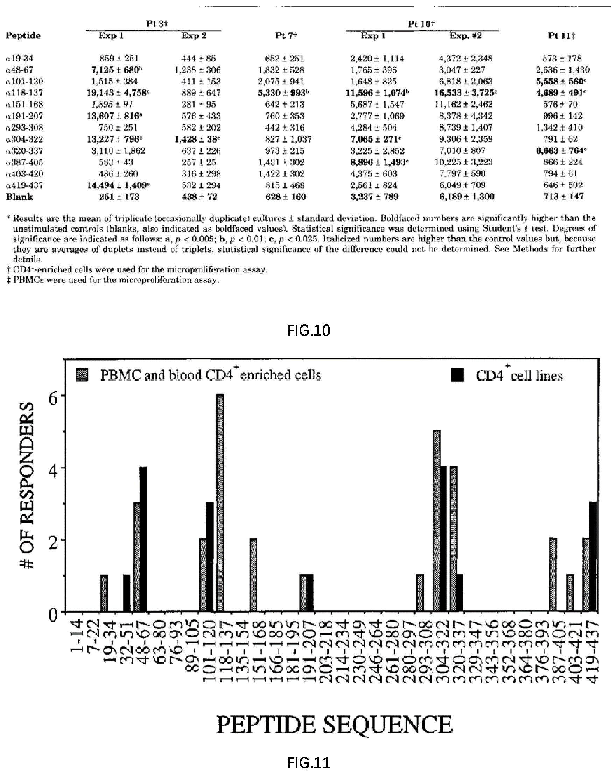

As shown in Figure 10 , the results depict the proliferation responses of four distinct MG patients (Pt 3/7/10/11) to different epitopes of the AChR α-subunit. Specifically, Pt 3/7/10 utilised CD4⁺-enriched lymphocytes, whilst Pt 11 employed peripheral blood mononuclear cells (PBMCs).The results demonstrate, on the one hand, shared epitopes eliciting responses across multiple patients: α118-137 induced significant proliferation in all four patients with exceptionally high intensity, representing the most central dominant epitope; α304-322 elicited significant proliferation in Pt3 and Pt10, also constituting an important dominant epitope. On the other hand, distinct individual variations in immune responses to AChR α-subunit epitopes were observed among MG patients: Pt 3 exhibited extremely potent proliferation against α48-67, while Pt 10 demonstrated significant proliferation against α387-405.

Figure 11: Light grey dotted columns indicate the frequency of recognition of different peptide epitopes on the α-subunit of AChR by fresh immune cells (PBMCs and CD4+ enriched cells) isolated from peripheral blood of seven MG patients. This shows that α118-137 has the highest number of responders and is the most widely recognised epitope in the peripheral blood of MG patients. α304-322 is also a high-frequency recognised epitope, Multiple responders were also observed for regions such as α48-67 and α387-405.Black bars denote recognition frequencies of these peptides in previously established long-term cultured anti-AChR CD4+ T cell lines derived from four MG patients. The tallest black bars correspond to α48-67 and α304-322, indicating these epitopes represent core targets universally recognised across all four long-term T cell lines. The black bar for α419-437, observed in approximately three cell lines, also represents a high-frequency recognised epitope within the cell lines.

Furthermore, to define the epitope repertoire of anti-AChR Th cells, this example examined the response of unsorted blood CD4⁺ cells and/or total lymphocytes from 22 MG patients to 20 amino acid overlapping synthetic peptides covering the entire sequence of the human muscle AChR α subunit.

The results demonstrated:
(1) Only patients with the most severe disease recognised α-subunit epitopes, predominantly young females;
(2) Removal of CD8⁺ cells is required for in vitro detection of AChR-specific CD4⁺ responses: when using CD8⁺ cell-depleted samples from two patients, distinct responses to multiple α-subunit peptide sequences were detectable, whereas their total peripheral blood mononuclear cell populations showed no reaction to any α-subunit peptide.
(3) The peptide recognition patterns of patients exhibit individual uniqueness, but the four most commonly recognised immunodominant regions in long-term AChR-specific CD4⁺ T cell lines or adjacent peptide sequences are: residues 48-67, 101-137, 293-337, and 408-437; followed by 89-105 and 320-337.

The key epitope sequences of the AChR α-subunit are presented in Table 16 below.

**Table 16. Key epitope sequences of the AChR α-subunit**

| S E Q I D N O . | Epitope | P os iti on | Reverse-translated cDNA sequence | S E Q I D N O . |
|---|---|---|---|---|
| 6 4 | EVNQIVTTNVRLKQ QWVDYNLK | 48 - 67 | GAAGTGAACCAGATTGTGACCACCAACGTGCGCCTGAAACAGCAGT GGGTGGATTATAACCTGAAA | 7 0 |
| 6 5 | RPDLVLYNNADGDF AIVKFTK | 89 - 10 5 | CGCCCGGATCTGGTGCTGTATAACAACGCGGATGGCGATTTTGCGA TTGTGAAATTTACCAAA | 7 1 |
| 6 6 | DFAIVKFTKVLLQYT | 10 1- | GATTTTGCGATTGTGAAATTTACCAAAGTGCTGCTGCAGTATACCGG | 7 2 |
| | GHITWTPPAIFKSYC EIIVTHFPFDE | 13 7 | CCATATTACCTGGACCCCGCCGGCGATTTTAAAAGCTATTGCGAAA TTATTGTGACCCATTTTCCGTTTGATGAA | |
| 6 7 | VIVINTHHRSPSTHV MPNWVRKVFIDTIPN IMFFSTMKRPSREKQ DKKI | 29 3-33 7 | GTGATTGTGATTAACACCCATCATCGCAGCCCGAGCACCCATGTGA TGCCGAACTGGGTGCGCAAAGTGTTTATTGATACCATTCCGAACATT ATGTTTTTTAGCACCATGAAACGCCCGAGCCGCGAAAAACAGGATA AAAAAATT | 7 3 |
| 6 8 | DTIPNIMFFSTMKRPS REKQDK | 32 0-33 7 | GATACCATTCCGAACATTATGTTTTTAGCACCATGAAACGCCCGAG CCGCGAAAAACAGGATAAA | 7 4 |
| 6 9 | MDHILLGVFMLVCII GTLAVFAGRLIELNQ QG | 40 8-43 7 | ATGGATCATATTCTGCTGGGCGTGTTTATGCTGGTGTGCATTATTGG CACCCTGGCGGTGTTTGCGGGCCGCCTGATTGAACTGAACCAGCAG GGC | 7 5 |

### (2) Selection of the MuSK Epitope

The full-length amino acid sequence of MuSK is shown in SEQ ID NO. 76. MuSK-Ig1 is located at positions 21-125 of said full-length amino acid sequence. This example provides an overview of the correlation between disease severity scores and MuSK-Ig1 epitope pattern reactivity in 22 Italian G-type patients. The correlation results, indicate a strong association between the MuSK-Ig1 epitope and disease severity scores, whereas other epitopes showed weaker correlations. This suggests that the Ig1 epitope is a primary driver of MG disease progression, thereby justifying its selection as the target epitope for mRNA immunotherapy.

The key peptide sequences within the MuSK-Ig1 domain, identified via NetMHCipan 4.1 screening in the IEDB, are presented in Table 17.

**Table 17. MuSK-Ig1 domain key peptide sequences**

| S E Q ID N O. | Epitope | Po siti on | Reverse-translated cDNA sequence | cD NA Seq uen ce Nu mb er |
|---|---|---|---|---|
| 7 | TEKLPKAPVITTPLE TVDALVEEVATFMC AVESYP | 21 - 55 | ACCGAAAAACTGCCGAAAGCGCCGGTGATTACCACCCCGCT GGAAACCGTGGATGCGCTGGTGGAAGAAGTGGCGACCTTTATGT GCGCGGTGGAAAGCTATCCG | 80 |
| 78 | QPEISWTRNKILIKLF DTRYSIRENGQLLTI LSVE | 56 - 88 | CAGCCGGAAATTAGCTGGACCCGCAACAAAATTCTGATTAA ACTGTTTGATACCCGCTATAGCATTCGCGAAAACGGCCAGCTGC TGACCATTCTGAGCGTGGAA | 81 |
| 79 | DSDDGIYCCTANNG VGGAVESCGALQV KMKPKITR | 89-12 3 | GATAGCGATGATGGCATTTATTGCTGCACCGCGAACAACGG CGTGGGCGGCGCGGTGGAAAGCTGCGGCGCGCTGCAGGTGAAA ATGAAACCGAAAATTACCCGC | 82 |

### (3) Preparation of mRNA

Furthermore, a complete cDNA sequence is constructed, wherein the cDNA comprises a 5'UTR (including the Kozak sequence), a coding region, and a 3'UTR. The nucleotide sequences of the 5'UTR and 3'UTR are identical to those in Example 6. The coding region encodes the target sequence and at least one epitope sequence from Tables 16-17, with multiple epitopes connected via linkers as part of the coding region. The leader sequence and linker sequence are as described in Example 6. In this example, the coding region of the cDNA sequence encompasses all reverse-translated cDNA sequences from Tables 16-17. The cDNA sequence of the coding region is shown as SEQ ID NO: 83. The coding region cDNA undergoes further codon optimisation using the method described in Example 6.The optimised coding region cDNA sequence is shown as SEQ ID NO. 84, with a GC content of 55.08%.

Furthermore, the optimised cDNA is transcribed into mRNA using the same method as in Example 6.

### 2. Preparation of LNPs

LNPs prepared using DSPC as the auxiliary lipid in Table 2 of Example 3, along with LNPs 1 and 7 (identical to Example 1), 22 and 26 from Table 4 of Example 5, were used.22 and 26 from Table 4 of Example 5 were loaded with the mRNA prepared in this example, designated as monovalent targeted LNPs, bivalent targeted LNPs, trivalent targeted LNPs, tetravalent targeted LNPs and pentavalent targeted LNPs respectively.

### 3. LNPs for the Treatment of MG

Using transgenic mouse models expressing HLA-DR3 and HLA-DQ8, MG mouse models were established either by adoptive transfer of AChR-reactive CD4⁺ T cells or direct immunisation with AChR.MG mouse models were treated with intravenous injections of monovalent, bivalent, trivalent, tetravalent, and pentavalent targeted LNPs, respectively, with the control group receiving blank LNPs. Therapeutic efficacy was assessed by measuring the proportion of Foxp3⁺ Tregs in spleen, levels of pro-inflammatory cytokines (IFN-γ, IL-17), circulating autoantibody concentrations, and clinical scores across groups. Results are presented in Table 18.

**Table 18: Proportion of Foxp3⁺ Tregs in Lymph Nodes, Levels of Pro-inflammatory Cytokines (IFN-γ, IL-17), Autoantibody Concentrations, and Clinical Scores in Each Group**

| Test item | Control | - targeted LNPs | Bivalent-targeted LNPs | Tertiary-Targeted LNPs | Quaternary Targeted LNPs | Pentavalent Targeted LNPs |
|---|---|---|---|---|---|---|
| Foxp3⁺ Treg proportion | 4.8 | 8.3 | 13.5 | 18.7 | 11.2 | 9.5 |
| IFN-γ (pg/mg) | 38.6 | 26.4 | 17.9 | 8.2 | 20.3 | 24.7 |
| IL-17 (pg/mg) | 34.2 | 23.8 | 15.4 | 7.5 | 18.6 | 22.1 |
| Autoantibody levels (ng/mg) | 156.3 | 112.7 | 78.5 | 39.8 | 89.4 | 105.2 |
| Clinical score (5 points) | 4.1 | 3.2 | 1.8 | 1 | 2.1 | 2.8 |

Table 18 results demonstrate that, compared with the control group, mice in the monovalent or multivalent targeted LNP groups exhibited increased Foxp3⁺ Tregs, reduced pro-inflammatory cytokines IFN-γ and IL-17, decreased autoantibody levels, and improved clinical scores. Among these, the trivalent targeted LNP group exhibited the most favourable outcomes across all assessed parameters, indicating superior capacity for inducing immune tolerance and optimal control of inflammatory responses. This effectively blocked autoantibody-mediated damage at the neuromuscular junction, with symptoms such as myasthenia gravis and ptosis substantially alleviated ( ), approaching near-normal levels.

Trivalent targeted LNPs synergistically target multiple receptors on the surface of LSECs. Simultaneous binding to these receptors significantly enhances the specific uptake efficiency by LSECs, thereby efficiently delivering antigenic epitopes. This induces substantial generation of Foxp3⁺ Tregs while simultaneously suppressing the activation of Th1 or Th17 cells, reducing the production of pro-inflammatory factors such as IFN-γ and IL-17, as well as autoantibodies.

### Example 10: Treatment of Toxic Diffuse Goitre

This example demonstrates that LNPs prepared in Example 1 can be employed for treating toxic diffuse goitre (Graves' disease).

Antigenic epitope peptide prediction, design, and mRNA preparation

The pathogenesis of GD involves an immune misrecognition where the thyroid follicular cell membrane's thyroid-stimulating hormone receptor (TSHR) is mistaken for a "foreign antigen," thereby inducing B lymphocytes to produce TSHR-specific autoantibodies. Additionally, an imbalance exists between the Th1 and Th2 helper T-cell (Th) subsets in patients. Hyperfunction of Th2 cells promotes B-cell activation and excessive autoantibody production through secreted cytokines (e.g., IL-4, IL-10). Concurrently, defective regulatory T-cell (Treg) function fails to suppress abnormal immune responses, exacerbating autoimmune disorder.

The amino acid sequence of TSHR is shown in SEQ ID NO: 85. Residues 1-412 of this sequence constitute the extracellular domain of TSHR, which serves as the primary target for autoantibodies. The frequency of HLA-DR3 in GD patients ranges from 40-55% (compared to 15-30% in the general population), with carriers exhibiting a 3-4-fold increased risk of developing the disease.

### (1) Investigation of TSHR extracellular domain binding affinity to HLA-DR3

Predictions of the binding affinity between the TSHR extracellular domain and HLA-DR3 were generated using the NIAID IEDB database ( ) and , with results presented in Table 19 below.

**Table 19. Predicted binding affinity results for TSHR extracellular domain epitopes with HLA-DR3**

| Allele | Start Position | Terminus | Length | Peptide | Percentage ranking |
|---|---|---|---|---|---|
| HLA-DRB1*03:01 | 79 | 93 | 15 | SRIYVSIDVTLQQLE | 4.50 |
| HLA-DRB1*03:01 | 78 | 92 | 15 | ISRIYVSIDVTLQQL | 4.70 |
| HLA-DRB1*03:01 | 81 | 95 | 15 | IYVSIDVTLQQLESH | 4.70 |
| HLA-DRB1*03:01 | 77 | 91 | 15 | NISRIYVSIDVTLQQ | 4.70 |
| HLA-DRB1*03:01 | 80 | 94 | 15 | RIYVSIDVTLQQLES | 4.70 |
| HLA-DRB1*03:01 | 6 | 20 | 15 | LLQLVLLLDLPRDLG | 6.50 |
| HLA-DRB1*03:01 | 7 | 21 | 15 | LQLVLLLDLPRDLGG | 6.50 |
| HLA-DRB1*03:01 | 8 | 22 | 15 | QLVLLLDLPRDLGGM | 6.50 |
| HLA-DRB1*03:01 | 137 | 151 | 15 | GLKMFPDLTKVYSTD | 6.80 |
| HLA-DRB1*03:01 | 136 | 150 | 15 | TGLKMFPDLTKVYST | 6.80 |
| HLA-DRB1*03:01 | 134 | 148 | 15 | FNTGLKMFPDLTKVY | 6.90 |
| HLA-DRB1*03:01 | 138 | 152 | 15 | LKMFPDLTKVYSTDI | 6.90 |
| HLA-DRB1*03:01 | 135 | 149 | 15 | NTGLKMFPDLTKVYS | 6.90 |
| HLA-DRB1*03:01 | 5 | 19 | 15 | DLLQLVLLLDLPRDL | 7.90 |
| HLA-DRB1*03:01 | 11 | 25 | 15 | LLLDLPRDLGGMGCS | 7.90 |
| HLA-DRB1*03:01 | 9 | 23 | 15 | LVLLLDLPRDLGGMG | 7.90 |
| HLA-DRB1*03:01 | 10 | 24 | 15 | VLLLDLPRDLGGMGC | 7.90 |

### (2) Investigation of the affinity between the extracellular domain of TSHR and H2-IAd/H2-IEd

Animal experiment epitope prediction based on the binding affinity between the TSHR extracellular domain and H2-IAd/H2-IEd yielded the results presented in Table 20 below.

**Table 20. Predicted binding affinity of TSHR extracellular domain epitopes with H2-IAd/H2-IEd**

| Allele | Start Point | Terminus | Length | Sequence | Peptide sequence | IC₅₀ | Percentage Sorting |
|---|---|---|---|---|---|---|---|
| H2-IAd | 233 | 247 | 15 | QTSVTALPS | VSQTSVTALPSKGLE | 30.24 | 0.06 |
| H2-IAd | 234 | 248 | 15 | QTSVTALPS | SQTSVTALPSKGLEH | 42.76 | 0.15 |
| H2-IAd | 232 | 246 | 15 | QTSVTALPS | DVSQTSVTALPSKGL | 47.29 | 0.22 |
| H2-IAd | 230 | 244 | 15 | QTSVTALPS | LLDVSQTSVTALPSK | 66.82 | 0.61 |
| H2-IAd | 231 | 245 | 15 | QTSVTALPS | LDVSQTSVTALPSKG | 74.75 | 0.84 |
| H2-IAd | 58 | 72 | 15 | ETHLRTIPS | KLIETHLRTIPSHAF | 84.33 | 0.99 |
| H2-IAd | 59 | 73 | 15 | ETHLRTIPS | LIETHLRTIPSHAFS | 98.80 | 1.40 |
| H2-IAd | 229 | 243 | 15 | QTSVTALPS | SLLDVSQTSVTALPS | 117.75 | 1.90 |
| H2-IAd | 57 | 71 | 15 | ETHLRTIPS | LKLIETHLRTIPSHA | 130.39 | 2.30 |
| H2-IAd | 60 | 74 | 15 | HLRTIPSHA | IETHLRTIPSHAFSN | 145.91 | 2.70 |
| H2-IAd | 161 | 175 | 15 | MTSIPVNAF | NPYMTSIPVNAFQGL | 156.97 | 3 |
| H2-IAd | 267 | 281 | 15 | LHLTRADLS | LSFLHLTRADLSYPS | 167.12 | 3.30 |
| H2-IAd | 56 | 70 | 15 | ETHLRTIPS | TLKLIETHLRTIPSH | 176.80 | 3.60 |
| H2-IAd | 266 | 280 | 15 | LHLTRADLS | SLSFLHLTRADLSYP | 180.64 | 3.80 |
| H2-IAd | 162 | 176 | 15 | MTSIPVNAF | PYMTSIPVNAFQGLC | 196.87 | 4.20 |
| H2-IAd | 235 | 249 | 15 | SVTALPSKG | QTSVTALPSKGLEHL | 198.96 | 4.30 |
| H2-IAd | 265 | 279 | 15 | LHLTRADLS | LSLSFLHLTRADLSY | 204.13 | 4.50 |
| H2-IAd | 100 | 114 | 15 | VTHIEIRNT | LSKVTHIEIRNTRNL | 266.99 | 6.30 |
| H2-IAd | 55 | 69 | 15 | ETHLRTIPS | QTLKLIETHLRTIPS | 312.34 | 7.80 |

Based on the results presented in Tables 19-20 above, a set of mRNA immunotherapy epitopes suitable for use in animal models (human TSHR immunisation) and patients has been identified, as shown in Table 21 below.

**Table 21: Preferred TSHR epitopes and their reverse-transcribed cDNA sequences**

| Allele | Position | Epitope with flanking sequences | SEQ ID NO. | Reverse Transcribed cDNA Sequence | SEQ ID NO. |
|---|---|---|---|---|---|
| HLA-DRB1*0 3:01 | 79-93 | NISRIYVSIDVT LQQLESH | 86 | AACATTAGCCGCATTTATGTGAGCATTGATGTG ACCCTGCAGCAGCTGGAAAGCCAT | 94 |
| | 7-21 | DLLQLVLLLDL PRDLGGMG | 87 | GATCTGCTGCAGCTGGTGCTGCTGCTGGATCTG CCGCGCGATCTGGGCGGCATGGGC | 95 |
| | 137-151 | NTGLKMFPDLT KVYSTDIF | 88 | AACACCGGCCTGAAAATGTTTCCGGATCTGAC CAAAGTGTATAGCACCGATATTTTT | 96 |
| H2-IAd | 234-248 | DVSQTSVTALP SKGLEHLK | 89 | GATGTGAGCCAGACCAGCGTGACCGCGCTGC CGAGCAAAGGCCTGGAACATCTGAAA | 97 |
| | 58-72 | TLKLIETHLRTI PSHAFSN | 90 | ACCCTGAAACTGATTGAAACCCATCTGCGCA CCATTCCGAGCCATGCGTTTAGCAAC | 98 |
| | 100-114 | YNLSKVTHIEIR NTRNLTY | 91 | TATAACCTGAGCAAAGTGACCCATATTGAAA TTCGCAACACCCGCAACCTGACCTAT | 99 |
| | 55-69 | STQTLKLIETHL RTIPSHA | 92 | AGCACCCAGACCCTGAAACTGATTGAAACCC ATCTGCGCACCATTCCGAGCCATGCG | 100 |
| | 267-281 | LSLSFLHLTRA DLSYPSHC | 93 | CTGAGCCTGAGCTTTCTGCATCTGACCCGCGC GGATCTGAGCTATCCGAGCCATTGC | 10 1 |

### (3) Preparation of mRNA

Furthermore, a complete cDNA sequence is constructed, wherein the cDNA comprises a 5'UTR (including the Kozak sequence), a coding region, and a 3'UTR. The nucleotide sequences of the 5'UTR and 3'UTR are identical to those in Example 6. The coding region encodes the target sequence and at least one epitope sequence from Table 21, with multiple epitopes connected via linkers as part of the coding region. The leader sequence and linker sequence are as described in Example 6. In this example, the coding region incorporates all epitopes from Table 21. The cDNA of the coding region is further subjected to codon optimisation using the method described in Example 6. The optimised coding region cDNA sequence is shown in SEQ ID NO: 102, with a GC content of 58.63%.

Furthermore, the optimised cDNA is transcribed into mRNA using the method described in Example 6.

### 2. Preparation of LNPs

LNPs prepared in Example 3 using DSPC as the auxiliary lipid in Table 2, along with those in Example 5 (Tables 4, 1 and 7 corresponding to Example 1),22 and 26 from Table 4 of Example 5 were loaded with the mRNA prepared in this example, designated as monovalent targeted LNPs, bivalent targeted LNPs, trivalent targeted LNPs, tetravalent targeted LNPs and pentavalent targeted LNPs respectively.

### 3. LNP Treatment of GD Disease

A GD mouse model was established as follows: Susceptible mouse strains (e.g., BALB/c) were repeatedly immunised via muscle electroporation using plasmid DNA encoding the TSHR α subunit (TSHR-289).

This induction method successfully elicits biologically active stimulating antibodies (TSAb). These antibodies bind to TSHR on thyroid cell surfaces and persistently activate downstream signalling pathways, leading to elevated thyroid hormone levels, diffuse thyroid enlargement, and thyroid follicular epithelial hyperplasia.

GD mouse models were treated with monovalent, bivalent, trivalent, tetravalent, and pentavalent targeted LNPs respectively, whilst the control group received blank LNPs. Therapeutic efficacy was assessed by measuring thyrotropin receptor antibodies (TRAb) levels, Th1/Th2 helper cell ratios, and IL-4/IL-10 concentrations in the blood of mice across all groups. Results are presented in Table 22.

**T able 22: Thyroid-stimulating hormone receptor antibodies (TRAb) levels, Th1/Th2 helper T-cell ratios, and IL-4/IL-10 levels in the blood of mice from each group**

| Test item | Control | Monovalent Targeted LNPs | Bivalent Targeted LNPs | Trivalent Targeted LNPs | Quaternary Targeted LNPs | Pentavalent Targeted LNPs |
|---|---|---|---|---|---|---|
| TRAb (ng/mg) | 8.9 | 6.7 | 4.2 | 3.5 | 5.1 | 6.3 |
| Th1/Th2 ratio | 0.3 | 0.6 | 0.9 | 1.1 | 0.8 | 0.5 |
| IL-4 (pg/mg) | 32.6 | 24.8 | 15.3 | 8.7 | 18.6 | 23.1 |
| IL-10 (pg/mg) | 4.2 | 9.5 | 18.3 | 27.6 | 15.7 | 10.2 |

As shown in Table 22, compared with the control group, mice in the monovalent or multivalent targeted LNP groups exhibited decreased TRAb levels, elevated Th1/Th2 ratios, reduced IL-4 levels, and increased IL-10 levels. Among these, mice in the trivalent targeted LNP group demonstrated the lowest TRAb and IL-4 levels, while exhibiting the highest Th1/Th2 ratio and IL-10 levels. This indicates effective blockade of TSHR antibody-mediated thyroid dysfunction, with the Th1/Th2 ratio approaching or even returning to the normal range. This corrected the characteristic Th2 hyperactivity observed in GD, while the reduced IL-4 levels significantly suppressed the pro-inflammatory function of Th2 cells. The increased IL-10 levels enhanced the anti-inflammatory immune response.

The trivalent targeted LNPs synergistically target multiple receptors on the surface of LSECs. Simultaneous binding to these receptors significantly enhances the specificity of LSEC uptake. Following efficient uptake of LNPs by LSECs, the mRNA-encoded TSHR optimal epitope is precisely delivered. This induces the proliferation of Foxp3⁺ Tregs, which secrete anti-inflammatory factors such as IL-10.while simultaneously suppressing Th2 cell activation, reducing IL-4 secretion and TRAb production, ultimately restoring immune homeostasis.

### Example 11: Treatment of Systemic Lupus Erythematosus

### 1. Prediction and Design of Antigenic Epitope Peptides and Preparation of mRNA

Systemic lupus erythematosus (SLE) is an autoimmune disorder arising from the combined effects of genetic susceptibility (e.g., HLA class II gene polymorphisms), environmental triggers (ultraviolet radiation, viral infections, etc.), and epigenetic modifications, which disrupt the body's immune tolerance; Its core mechanism involves abnormal B-cell activation producing various autoantibodies, including antinuclear antibodies. The resulting immune complexes deposit in multiple organ tissues such as skin, kidneys, and joints. This is accompanied by T-cell subset dysregulation (e.g., Treg dysfunction, Th17 cell hyperproliferation), triggering persistent inflammation and tissue damage, ultimately leading to multisystem involvement. Histones (classified into five types: H1, H2A, H2B, H3, H4), SNRNP70 (P140), SMD1, SMD3, U1A, and U1C are all core autoantigens implicated in SLE pathogenesis.

This embodiment employs the NetMHCipan_el 4.1 server method to screen for optimal epitopes of the aforementioned antigens and converts these optimal epitopes into reverse-transcribed cDNA sequences, as shown in Table 23 below.

**Table 23: Preferred epitopes for different antigens**

| Serial No. | Epitope | Position | Sequence | SEQ ID No. | Reverse Transcribed cDNA Sequence | SEQ ID No. |
|---|---|---|---|---|---|---|
| 1 | Histone H1 | 22-42 | STDHPKYSDMI VAAIQAEKNR | 103 | AGCACCGATCATCCGAAATATAGCGATATGATTG TGGCGGCGATTCAGGCGGAAAAAAACCGC | 113 |
| 2 | Histone H3 | 82-105 | DLRFQSSAVMA LQEACEAYLVG LF | 104 | GATCTGCGCTTTCAGAGCAGCGCGGTGATGGCGC TGCAGGAAGCGTGCGAAGCGTATCTGGTGGGCC TGTTT | 114 |
| 3 | Histone H3 | 115-135 | KRVTIMPKDIQL ARRIRGERA | 105 | AAACGCGTGACCATTATGCCGAAAGATATTCAG CTGGCGCGCCGCATTCGCGGCGAACGCGCG | 115 |
| 4 | Histone H4 | 16-32 | KRHRKVLRDNI QGITKP | 106 | AAACGCCATCGCAAAGTGCTGCGCGATAACAT TCAGGGCATTACCAAACCG | 116 |
| 5 | Histone H4 | 71-94 | TYTEHAKRKTV AMDVVYALKR QG | 107 | ACCTATACCGAACATGCGAAACGCAAAACCGT GGCGATGGATGTGGTGTATGCGCTGAAACGCC AGGGC | 117 |
| 6 | SNRN P70 (P140) | 131-151 | RIHMVYSKRSG KPRGYAFIEY | 108 | CGCATTCATATGGTGTATAGCAAACGCAGCGG CAAACCGCGCGGCTATGCGTTTATTGAATAT | 118 |
| 7 | SMD1 | 32-46 | VDVSMNTHLK AVKMT | 109 | GTGGATGTGAGCATGAACACCCATCTGAAAGC GGTGAAAATGACC | 119 |
| 8 | SMD3 | 57-71 | QLEQVVYIRGSKI RFL | 110 | CAGCTGGAACAGGTGTATATTCGCGGCAGCAA AATTCGCTTTCTG | 120 |
| 9 | U1A | 136-151 | AVQGPVPGMPP | 111 | GCGGTGCAGGGCCCGGTGCCGGGCATGCCGCC | 121 |
| | | | MTQAP | | GATGACCCAGGCGCCG | |
| 10 | U1C | 63-76 | PPTPFSAPPPAG AM | 112 | CCGCCGACCCCGTTTAGCGCGCCGCCGCCGGC GGGCGCGATG | 122 |

Furthermore, a complete cDNA sequence is constructed, wherein the cDNA comprises a 5'UTR (including a Kozak sequence), a coding region, and a 3'UTR. The nucleotide sequences of the 5'UTR and 3'UTR are identical to those in Example 6. The coding region encodes the target sequence and at least one epitope sequence from Table 23, with multiple epitopes connected via linkers as part of the coding region. The leader sequence and linker sequence are as described in Example 6. In this example, two cDNA sequences were designed based on the epitope sequences in Table 23. The codons in the coding region cDNA were further optimised using the method described in Example 6. One of the optimised coding region cDNA sequences, as shown in SEQ ID NO. 123, contains the preferred epitope sequence for P140 and has a GC content of 54.59%.The other optimised coding region cDNA sequence is shown as SEQ ID NO. 124, incorporating all preferred epitopes from Table 23 with a GC content of 57.48%.

Furthermore, the optimised cDNA was transcribed into mRNA using the method described in Example 6.

### 2. Preparation of LNPs

The LNPs prepared using DSPC as the auxiliary lipid in Table 2 of Example 3, along with LNPs 1 and 7 (identical to Example 1) and LNPs 22 and 26 from Table 4 of Example 5,22 and 26 from Table 4 of Example 5, respectively, were loaded with the mRNA prepared in this example (coding sequence as shown in SEQ ID NO. 124), designated as monovalent targeted LNPs, bivalent targeted LNPs, trivalent targeted LNPs, tetravalent targeted LNPs and pentavalent targeted LNPs respectively.

### 3. LNP Therapy for SLE

The Fas^{lpr}mice represent a classical SLE mouse model, exhibiting MRL-Ipr/lymphoproliferative characteristics. This Fas^{lpr}mouse model is a spontaneous animal model arising from a spontaneous mutation in the Fas gene, which disrupts the Fas-mediated apoptosis pathway. Consequently, auto-reactive lymphocytes that should be eliminated via apoptosis accumulate extensively within the organism. Pathologically, LPR mice exhibit marked lymphadenopathy, splenomegaly, and produce high-titre autoantibodies such as anti-dsDNA, ultimately triggering severe immune complex glomerulonephritis. As this model paradigmatically reflects autoimmune damage resulting from peripheral immune tolerance loss, it is widely employed to evaluate novel immunotherapies (such as mRNA vaccines inducing regulatory T cell expansion) in restoring immune homeostasis and reversing inflammatory responses.

Validation of the successful Fas^{lpr}mouse model construction: Firstly, single-cell RNA sequencing was employed to detect differences in mRNA expression of inflammation-related genes between lupus patients and healthy volunteers. Results are shown), where scRNA-Seq data reveal extensive upregulation of inflammatory genes in lupus patients compared to healthy volunteers (yellow, orange, and red indicate increased gene expression; purple or blue indicate decreased expression).Further comparison of mRNA expression differences in inflammation-related genes between Fas^{lpr}mouse models and wild-type mice , revealing that Fas^{lpr}mouse models exhibit a similar pattern of inflammatory gene expression to human patients.

Sixteen-week-old Fas^{lpr}mice with severe SLE received intravenous injections of three doses of monovalent targeted LNPs, bivalent targeted LNPs, trivalent targeted LNPs, tetravalent targeted LNPs, pentavalent targeted LNPs, and blank LNPs (control), administered every three weeks. Following the third injection, mice were monitored for a further 10 weeks (i.e., weeks 16-26). Monitoring parameters included: mouse survival rates from weeks 16-26; analysis of pro-inflammatory cytokines in peripheral blood at week 16; and single-cell RNA sequencing of blood, bone marrow, kidney, and spleen tissues from mice euthanised after week 26.

The survival rate of mice from weeks 16 to 26 is presented in Table 24 below.

**Table 24: Survival rate of mice at 16-26 weeks post-treatment**

| Time | Mouse Survival Rate (%) | | | | | |
|---|---|---|---|---|---|---|
| | Control | Monovalent Targeted LNPs | - targeted LNPs | Trivalent-targeted LNPs | Quadrivalent-targeted LNPs | Pentavalent targeted LNPs |
| Week 16 | 100 | 100% | 100% | 100% | 100% | 100% |
| Week 18 | 85 | 86% | 95% | 98% | 90% | 88% |
| Week 20 | 50 | 65% | 80% | 90% | 75% | 70% |
| Week 22 | 40 | 58% | 72% | 85% | 68% | 63% |
| Week 26 | 25 | 45% | 65% | 78% | 58% | 52% |

As shown in Table 24, the survival rate of mice in the control group at week 26 was only 25%. Following treatment with monovalent or multivalent LNPs, the survival rate at week 26 increased across all groups, with the highest survival rate observed in mice treated with trivalent targeted LNPs.

Analysis of pro-inflammatory cytokines at week 16 revealed that, compared to the control group, mice injected with monovalent targeted LNPs, bivalent targeted LNPs, trivalent targeted LNPs, tetravalent targeted LNPs, and pentavalent targeted LNPs exhibited reduced numbers of neutrophils, monocytes, and dendritic cells in their blood. However, the reduction in these inflammatory cells was most pronounced in mice treated with trivalent targeted LNPs.

At 26 weeks post-injection, mice were euthanised and blood, bone marrow, kidney, and spleen samples were collected for single-cell RNA sequencing. Results demonstrated reduced expression of inflammatory genes in blood, bone marrow, kidney, and spleen tissues of mice injected with monovalent, bivalent targeted LNPs, trivalent targeted LNPs, tetravalent targeted LNPs, and pentavalent targeted LNPs exhibited reduced expression of inflammatory genes in blood, bone marrow, kidney, and spleen tissues. The most pronounced reduction in inflammatory gene expression was observed in the trivalent targeted LNPs ( ) group.

### Example 12: Treatment of Primary Biliary Cholangitis

### 1. Prediction and design of antigenic epitope peptides, and preparation of mRNA

The core autoantigen in primary biliary cholangitis (PBC) is the E2 subunit of the pyruvate dehydrogenase complex (PDC-E2). During normal apoptosis, PDC-E2 undergoes glutathionyl modification; the modified PDC-E2 is then encapsulated within apoptotic bodies. These apoptotic bodies containing modified PDC-E2 are phagocytosed by antigen-presenting cells (APCs). APCs present PDC-E2 via major histocompatibility complex molecules (MHC-I/MHC-II) to immune cells, triggering specific immune responses including humoral and cellular immunity. Upon recognising PDC-E2, B lymphocytes differentiate into plasma cells, secreting anti-PDC-E2 antibodies (specifically the AMA-M2 subtype, the hallmark antibody of PBC). These antibodies bind to PDC-E2 to form immune complexes, which can deposit on cholangiolar epithelium and activate the complement system (e.g., C3, C5), further amplifying the inflammatory response.CD4+ Th cells, upon activation by MHC-II-presented antigens, differentiate into Th1, Th17, and other subsets, secreting cytokines (such as IFN-γ, TNF-α, IL-17). These cytokines not only directly damage cholangiocytes but also recruit additional immune cells (e.g., macrophages) to the site.CD8+ CTLs, upon activation by MHC-I-presented antigens, directly recognise and eliminate bile duct epithelial cells expressing PDC-E2 (bile duct cells themselves also express PDC-E2), causing specific apoptosis of these cells.

HLA-DRB4*01:01 is a specific allele of the HLA-DRB4 gene, which encodes part of the HLA-DR molecule-an MHC class II protein involved in immune responses. In PBC, HLA-DRB4*01:01 plays a crucial role, as certain HLA-DR alleles are associated with increased susceptibility to autoimmune diseases. Research indicates that specific HLA-DRB4 alleles, particularly HLA-DRB4*01:01, are associated with an increased risk of PBC development. Expression of this allele predisposes individuals to an autoimmune response targeting the PDC-E2 antigen, a key autoantigen in the pathogenesis of PBC. The HLA-DRB4 gene plays a pivotal role in presenting PDC-E2 protein peptides to CD4⁺ T cells. In PBC, PDC-E2 serves as one of the primary autoantigens, and the HLA-DRB4*01:01 allele may present specific epitopes of PDC-E2, thereby promoting the immune activation and chronic inflammation observed in PBC. Multiple genetic studies support the association between HLA-DRB4 alleles and PBC. Detection rates of HLA-DRB4*01:01 are higher in PBC patients compared to healthy individuals, particularly among those harbouring anti-mitochondrial antibodies (AMA), a key diagnostic marker for PBC.

This embodiment employs the IEDB database to predict the dihydrotiobis(thio)octanamide acyltransferase (DLAT) epitope for PDC, whose amino acid sequence is shown in SEQ ID NO. 125 (residues 1-86 constitute the mitochondrial transport peptide).The target binding partner for the selected epitope peptide is human HLA-DR, with the allele HLA-DRB4*01:01 chosen. The epitope peptide length is 15 amino acids. The epitope peptide screening was conducted using the NetMHCipan 4.1 server's prediction parameters. Epitopes exhibiting high affinity and located within antigenic conserved regions were selected. The prediction results are presented in Table 25 below.

**Table 25. Predicted affinity results for DLAT epitopes with HLA-DRB4*01:01**

| Allele | Start Position | End Position | Length | Core Sequence | Peptide Sequence | Score | Percentile Rank |
|---|---|---|---|---|---|---|---|
| HLA-DRB4*01:01 | 122 | 136 | 15 | IAEVETDKA | GDLIAEVETDKATVG | 0.8970 | 0.08 |
| HLA-DRB4*01:01 | 249 | 263 | 15 | LAEIETDKA | GDLLAEIETDKATIG | 0.8774 | 0.10 |
| HLA-DRB4*01:01 | 121 | 135 | 15 | IAEVETDKA | EGDLIAEVETDKATV | 0.8606 | 0.11 |
| HLA-DRB4*01:01 | 248 | 262 | 15 | LAEIETDKA | EGDLLAEIETDKATI | 0.8242 | 0.14 |
| HLA-DRB4*01:01 | 123 | 137 | 15 | IAEVETDKA | DLIAEVETDKATVGF | 0.7718 | 0.19 |
| HLA-DRB4*01:01 | 120 | 134 | 15 | IAEVETDKA | NEGDLIAEVETDKAT | 0.7772 | 0.19 |
| HLA-DRB4*01:01 | 247 | 261 | 15 | LAEIETDKA | SEGDLLAEIETDKAT | 0.7443 | 0.23 |
| HLA-DRB4*01:01 | 250 | 264 | 15 | LAEIETDKA | DLLAEIETDKATIGF | 0.6772 | 0.32 |
| HLA-DRB4*01:01 | 311 | 325 | 15 | LKPQVPPPT | VTDLKPQVPPPTPPP | 0.4915 | 0.76 |
| HLA-DRB4*01:01 | 594 | 608 | 15 | LVPADNEKG | EDKLVPADNEKGFDV | 0.4510 | 0.93 |
| HLA-DRB4*01:01 | 428 | 442 | 15 | VIAQRLMQS | IRRVIAQRLMQSKQT | 0.3957 | 1.20 |
| HLA-DRB4*01:01 | 593 | 607 | 15 | LVPADNEKG | SEDKLVPADNEKGFD | 0.3968 | 1.20 |
| HLA-DRB4*01:01 | 310 | 324 | 15 | LKPQVPPPT | EVTDLKPQVPPPTPP | 0.4005 | 1.20 |
| HLA-DRB4*01:01 | 427 | 441 | 15 | VIAQRLMQS | NIRRVIAQRLMQSKQ | 0.3598 | 1.50 |
| HLA-DRB4*01:01 | 534 | 548 | 15 | IANDVVSLA | VETIANDVVSLATKA | 0.3473 | 1.60 |
| HLA-DRB4*01:01 | 531 | 545 | 15 | VETIANDVV | IKGVETIANDVVSLA | 0.3223 | 1.90 |
| HLA-DRB4*01:01 | 119 | 133 | 15 | IAEVETDKA | INEGDLIAEVETDKA | 0.3079 | 2 |

Based on the affinity binding results and data in Table 25 (J Exp Med (1995) 181 (5): 1835-1845; J Clin Invest. 1998;102(10):1831-1840; Journal of Autoimmunity 149 (2024) 103327), the following preferred epitopes in Table 26 were selected.

**Table 26. Preferred epitopes for DLAT**

| Number | Start-End | Sequence | SEQ NO ID. | Reverse-translated cDNA sequence | SEQ NO ID. |
|---|---|---|---|---|---|
| 1 | 121-140 | WEKKEGDKINEGDLIAE VETDKATVGFESLEE | 126 | TGGGAAAAAAAGAAGGCGATAAAATTAACGA AGGCGATCTGATTGCGGAAGTGGAAACCGATAA AGCGACCGTGGGCTTTGAAAGCCTGGAAGAA | 132 |
| 2 | 241-268 | EKKVGEKLSEGDLLAEI ETDKATIGFEVQEEG | 127 | GAAAAAAAAGTGGGCGAAAAACTGAGCGAAGG CGATCTGCTGGCGGAAATTGAAACCGATAAAGC GACCATTGGCTTTGAAGTGCAGGAAGAAGGC | 133 |
| 3 | 425-444 | IPISNIRRVIAQRLMQSK QTIPHY | 128 | ATTCCGATTAGCAACATTCGCCGCGTGATTG CGCAGCGCCTGATGCAGAGCAAACAGACCATTC CGCATTAT | 134 |
| 4 | 531-548 | AHIKGVETIANDVVSLA TKARE | 129 | GCGCATATTAAAGGCGTGGAAACCATTGCGAAC GATGTGGTGAGCCTGGCGACCAAAGCGCGCGAA | 135 |
| 5 | 311-325 | PTEVTDLKPQVPPPTPPP VA | 130 | CCGACCGAAGTGACCGATCTGAAACCGCAGGTG CCGCCGCCGACCCCGCCGCCGGTGGCG | 136 |
| 6 | 593-607 | GASEDKLVPADNEKGF DVA | 131 | GGCGCGAGCGAAGATAAACTGGTGCCGGCGGAT AACGAAAAAGGCTTTGATGTGGCG | 137 |

Furthermore, a complete cDNA sequence is constructed, wherein the cDNA comprises a 5'UTR (including the Kozak sequence), a coding region, and a 3'UTR. The nucleotide sequences of the 5'UTR and 3'UTR are identical to those in Example 6. The coding region encodes the target sequence and at least one epitope sequence from Table 26, with multiple epitopes connected via linkers as part of the coding region. The leader sequence and linker sequence are as described in Example 6. In this embodiment, the coding region of the cDNA sequence comprises all reverse-translated cDNA sequences from Table 26. The sequence of the coding region is as shown in SEQ ID NO: 138. The cDNA of the coding region is further optimised for codon efficiency using the method described in Example 6.The optimised coding region cDNA sequence is shown in SEQ ID NO. 139, with a GC content of 59.87%.

Furthermore, the optimised cDNA was transcribed into mRNA using the same method as in Example 6.

### 2. Preparation of LNPs

LNPs prepared using DSPC as the auxiliary lipid in Table 2 of Example 3, along with those from entries 1 and 7 (identical to Example 1), 22 and 26 in Table 4 of Example 5, were used.22 and 26 from Table 4 of Example 5 were loaded with the mRNA prepared in this example, designated as monovalent targeted LNPs, bivalent targeted LNPs, trivalent targeted LNPs, tetravalent targeted LNPs and pentavalent targeted LNPs respectively.

### 3. LNP Therapy for PBC

The 2-OA-PBC model is a classical experimental animal model established by mimicking the immunopathological characteristics of primary biliary cholangitis (PBC). This model is typically generated by using 2-octynoic acid-modified ovalbumin (2-OA-OVA) as the core antigen, combined with specific immunological adjuvants (such as Alhydrogel or high-affinity nanoadjuvants) to induce systemic sensitisation in mice. Repeated subcutaneous injections of the sensitising antigen induce an autoimmune response directed against bile duct epithelial components, resulting in marked lymphocytic infiltration around hepatic bile ducts and damage to small bile ducts. This model effectively mimics the pathophysiological processes observed in PBC patients, including the hyperactive Th1/Th17 immune response and impaired regulatory T cell (Treg) function.

Intravenous administration of three doses of monovalent targeted LNPs, bivalent targeted LNPs, trivalent targeted LNPs, tetravalent targeted LNPs, pentavalent targeted LNPs, and blank LNPs (control) to the PBC mouse model, administered every three weeks. Following the third injection, mice were monitored continuously for a further 10 weeks (i.e., weeks 16-26). Survival rates were recorded from weeks 16 to 26. At week 26, mice from each group were euthanised, and their livers were harvested to determine the proportion of Tregs and assess infiltration of inflammatory cytokines TNF-α, IFN-γ, and IL-17.

The survival rates of mice from weeks 16 to 26 are presented in Table 27 below.

**Table 27: Survival rate of mice from weeks 16 to 26 post-treatment**

| Time | Mouse Survival Rate (%) | | | | | |
|---|---|---|---|---|---|---|
| | Control | Monovalent Targeted LNPs | Bivalent Targeted LNPs | Trivalent Targeted LNPs | Quaternary-targeted LNPs | Pentavalent Targeted LNPs |
| Week 16 | 100% | 100% | 100% | 100% | 100% | 100% |
| Week 18 | 88% | 90% | 96% | 99% | 93% | 91% |
| Week 20 | 62% | 70% | 85% | 92% | 78% | 73% |
| Week 22 | 45% | 58% | 76% | 88% | 69% | 65% |
| Week 26 | 30% | 48% | 68% | 82% | 60% | 55% |

As shown in Table 27, the survival rate of mice in the control group at week 26 was only 30%. Following treatment with monovalent or multivalent LNPs , the survival rate at week 26 increased in all groups, with the highest survival rate observed in mice treated with trivalent targeted LNPs.

The proportion of Foxp3⁺ Tregs in the livers and the infiltration levels of inflammatory cytokines TNF-α, IFN-γ, and IL-17 in mice from each group after euthanasia are presented in Table 28.

**Table 28: Proportion of Tregs in the Liver and Infiltration of Inflammatory Cytokines TNF-α, IFN-γ, and IL-17**

| Test item | Control | Monovalent Targeted LNPs | Bivalent Targeted LNPs | Trivalent Targeted LNPs | Quaternary Targeted LNPs | Pentavalent Targeted LNPs |
|---|---|---|---|---|---|---|
| Foxp3⁺ Treg proportion (%) | 5.3 | 8.7 | 12.4 | 15.7 | 10.2 | 9.1 |
| TNF-α (pg/mg) | 42.6 | 31.8 | 20.5 | 9.8 | 24.7 | 28.3 |
| IFN-γ (pg/mg) | 38.4 | 27.5 | 17.2 | 8.3 | 21.6 | 25.1 |
| IL-17 (pg/mg) | 35.7 | 25.3 | 15.8 | 7.6 | 19.4 | 22.8 |

As shown in Table 22, compared with the control group, mice in the monovalent or multivalent targeted LNP groups exhibited increased proportions of Foxp3⁺ Tregs and reduced levels of TNF-α, IFN-γ, and IL-17. Among these, the trivalent targeted LNP group demonstrated the highest proportion of Foxp3⁺ Tregs, indicating the strongest capacity for inducing immune tolerance; while exhibiting the lowest levels of TNF-α, IFN-γ, and IL-17, indicating effective control of hepatic inflammatory infiltration.

The trivalent targeted LNPs synergistically targeted multiple receptors on the surface of LSECs. Simultaneous binding to multiple receptors significantly enhanced the specific uptake efficiency of LSECs. Following efficient uptake of LNPs by LSECs, precise presentation of antigenic epitopes occurred. This process induced substantial proliferation of Foxp3⁺ Tregs, which secreted anti-inflammatory factors to suppress abnormal immune responses; while simultaneously suppressing Th1/Th17 cell activation. This reduces the release of pro-inflammatory factors such as TNF-α, IFN-γ, and IL-17, thereby diminishing bile duct epithelial cell damage and immune complex deposition. Consequently, mouse survival time is extended and hepatic pathological conditions are ameliorated.

### Example 13: Treatment of Multiple Sclerosis

### 1. Prediction and Design of Antigenic Epitope Peptides and Preparation of mRNA

Multiple sclerosis (MS) is a chronic autoimmune disorder in which the body's own T cells attack myelin in the central nervous system (CNS), leading to demyelination of nerve fibres and neurodegeneration.

This embodiment selects three core proteins of myelin, including human MBP protein (amino acid sequence as shown in SEQ ID NO.140),human MOG protein (amino acid sequence as shown in SEQ ID NO. 141), and human PLP protein (amino acid sequence as shown in SEQ ID NO. 142). Using the NetMHCipan_el 4.1 server, the binding of epitope peptides from these three core proteins to MHC-II was predicted. Epitopes with high affinity and located within antigenic conserved regions were selected. with the optimised epitope results presented in Table 29 below.

**Table 29. Preferred epitope screening results for MBP, MOG, and PLP**

| Site/Peptide Name | Protein Region | Amino Acid Sequence | SEQ ID NO. | MHC Restriction |
|---|---|---|---|---|
| MBP1 | 28-46 | FLPRHRDTGILDSIGRFFG | 143 | HLA-DQ6 |
| MBP2 | 129-147 | GGRASDYKSAHKGFKGVDA | 144 | HLA-DQ6 |
| MBP3 | 138-156 | HKGFKGVDAQGTLSKIFKL | 145 | DR2a/DR2b |
| MBP4 | 81-101 | QDENPVVHFFKNIVTPRTPPP | 146 | DR2b |
| MOG1 | 1-20 | GQFRVIGPRHPIRALVGDEV | 147 | HLA-DRB1*15:01, *04:01 |
| MOG2 | 35-55 | MEVGWYRPPFSRVVHLYRNGK | 148 | HLA-DRB1*15:01 |
| MOG3 | 96-110 | GGFTCFFRDHSYQEEAAME | 149 | HLA-DRB1*04:01 |
| PLP1 | 95-116 | AVRQIFGDYKTTICGKGLSATV | 150 | HLA-DRB1*1501, HLA-DR2, DR4 |

Furthermore, the reverse-translated cDNA sequences for the eight preferred epitopes listed in Table 29 are shown in Table 30 below.

**Table 30. Reverse-translated cDNA sequences for eight preferred epitopes**

| Antigenic Epitope Name | Site | cDNA Sequence | SEQ ID NO. |
|---|---|---|---|
| MBP1 | 28-46 | TTTCTGCCGCGCCATCGCGATACCGGCATTCTGGATAGCATTGGCCG CTTTTTTGGC | 151 |
| MBP2 | 129-147 | GGCGGCCGCGCGAGCGATTATAAAAGCGCGCATAAAGGCTTTAAAG GCGTGGATGCG | 152 |
| MBP3 | 138-156 | CATAAAGGCTTTAAAGGCGTGGATGCGCAGGGCACCCTGAGCAAAA TTTTTAAACTG | 153 |
| MBP4 | 81-101 | CAGGATGAAAACCCGGTGGTGCATTTTTTAAAAACATTGTGACCCC GCGCACCCCGCCGCCG | 154 |
| MOG1 | 1-20 | GGCCAGTTTCGCGTGATTGGCCCGCGCCATCCGATTCGCGCGCTGGT GGGCGATGAAGTG | 155 |
| MOG2 | 35-55 | ATGGAAGTGGGCTGGTATCGCCCGCCGTTTAGCCGCGTGGTGCATCT GTATCGCAACGGCAAA | 156 |
| MOG3 | 96-110 | GGCGGCTTTACCTGCTTTTTCGCGATCATAGCTATCAGGAAGAAGC GGCGATGGAA | 157 |
| PLP1 | 95-116 | GCGGTGCGCCAGATTTTGGCGATTATAAAACCACCATTTGCGGCAA AGGCCTGAGCGCGACCGTG | 158 |

Furthermore, a complete cDNA sequence is constructed, wherein the cDNA comprises a 5'UTR (including a Kozak sequence), a coding region, and a 3'UTR. The nucleotide sequences of the 5'UTR and 3'UTR are identical to those in Example 6. The coding region encodes the target sequence and at least one epitope sequence from Table 30, with multiple epitopes connected via linkers as part of the coding region. The leader sequence and linker sequence are as described in Example 6. In this embodiment, the coding region of the cDNA sequence comprises all reverse-translated cDNA sequences from Table 30. The coding region cDNA undergoes further codon optimisation using the method described in Example 6. The optimised coding region cDNA sequence is shown in SEQ ID NO: 159, with a GC content of 60.37%.

Furthermore, the optimised cDNA is transcribed into mRNA using the method described in Example 6.

### 2. Preparation of LNPs

LNPs prepared using DSPC as the auxiliary lipid in Table 2 of Example 3, along with those from entries 1 and 7 (identical to Example 1), 22 and 26 in Table 4 of Example 5, were utilised. These LNPs were designated as monovalent targeted LNPs, bivalent targeted LNPs, trivalent targeted LNPs, and tetravalent targeted LNPs, respectively.22 and 26 from Table 4 of Example 5 were loaded with the mRNA prepared in this example, designated as monovalent targeted LNPs, bivalent targeted LNPs, trivalent targeted LNPs, tetravalent targeted LNPs and pentavalent targeted LNPs respectively.

### 3. LNP Therapy for MS

Myelin Oligodendrocyte Glycoprotein (MOG)-induced experimental autoimmune encephalomyelitis (EAE) stands as one of the most widely employed animal models. MOG constitutes a pivotal antigen located within the outermost layer of myelin sheaths in the central nervous system, exhibiting potent immunogenicity capable of inducing specific immune attacks against neural myelin. In establishing the mouse model of MS, mice were subcutaneously immunised with a mixture of the MOG(35-55) peptide fragment and complete Freund's adjuvant (CFA), supplemented with an injection of pae - dihydrocorytoleptin (PTX) to enhance blood-brain barrier permeability. This model highly mimics core pathological features of MS, including substantial inflammatory cell infiltration within the central nervous system, extensive demyelinating lesions, and consequent progressive limb paralysis. As the immune response in this model is primarily driven by Th1 and Th17 cells alongside impaired Treg function, it has become a core system for evaluating the immune tolerance-inducing capacity of mRNA-LNP platforms. Targeted hepatic delivery of mRNA encoding MOG epitopes effectively induces systemic expansion of antigen-specific Tregs, thereby suppressing effector T cell attacks on the nervous system and restoring immune homeostasis.

MS mouse models received intravenous injections of monovalent, bivalent, trivalent, tetravalent, and pentavalent targeted LNPs respectively, administered every three weeks for a total of three doses. The control group received blank LNPs. Two weeks after the final injection, peripheral blood was collected to measure autoantibody levels. Mice were then euthanised for spleen harvesting to assess Foxp3⁺ Treg proportion, central nervous tissue sampling to determine IFN-γ, IL-17A, and TNF-α levels, and nerve fibre damage scoring. Results are presented in Table 31.

**Table 31: Peripheral blood autoantibody levels, splenic Foxp3⁺ Treg proportion, central tissue IFN-γ, IL-17A and TNF-α levels, and nerve fibre damage scores in each group**

| Test item | Control | Monovalent Targeted LNPs | Bivalent-targeted LNPs | Trivalent Targeted LNPs | Quaternary Targeted LNPs | Pentavalent Targeted LNPs |
|---|---|---|---|---|---|---|
| Foxp3⁺ Tregs (%) | 5.1 | 8.9 | 13.4 | 16.8 | 11.3 | 9.7 |
| IFN-γ (pg/mg) | 41.7 | 30.2 | 18.6 | 8.5 | 22.8 | 27.5 |
| IL-17A (pg/mg) | 37.5 | 26.4 | 16.3 | 7.9 | 20.1 | 24.3 |
| TNF-α (pg/mg) | 39.2 | 28.7 | 17.5 | 8.1 | 21.6 | 25.8 |
| Autoantibody levels (ng/mL) | 162.4 | 118.7 | 76.3 | 40.5 | 89.6 | 105.2 |
| Nerve fibre damage score | 4.3 | 3.4 | 1.9 | 0.8 | 2.3 | 2.9 |

As shown in Table 31, compared with the control group, mice in the monovalent or multivalent targeted LNP groups exhibited reduced autoantibody levels, increased Foxp3⁺ Treg proportion, decreased IFN-γ, IL-17A and TNF-α content, and lower nerve fibre damage scores. Among these, the trivalent targeted LNP group demonstrated the lowest autoantibody levels, highest Foxp3⁺ Treg proportion, the lowest IFN-γ, IL-17A, and TNF-α levels, and the lowest nerve fibre damage scores. This indicates the strongest capacity to induce antigen-specific immune tolerance, with highly effective control of inflammatory infiltration in the central nervous system and significant alleviation of myelin loss and axonal damage, approaching normal levels.

The trivalent targeted LNPs synergistically targeted multiple receptors on the surface of LSECs. Simultaneous binding to multiple receptors substantially enhanced the specific uptake efficiency of LSECs. Following efficient uptake of LNPs by LSECs, on the one hand, they induced substantial proliferation of Foxp3⁺ Tregs, which secreted anti-inflammatory factors to suppress the activation of autoreactive T cells; while simultaneously inhibiting Th1/Th17 cells from secreting pro-inflammatory factors such as IFN-γ and IL-17A, thereby reducing autoantibody production. This mechanism blocks myelin loss and nerve fibre damage, improving the pathological progression of MS.

### Example 14: Treatment of Sjögren's Syndrome

### 1. Prediction and Design of Antigenic Epitope Peptides and Preparation of mRNA

Sjögren's syndrome (SS) is an autoimmune disorder arising from the combined disruption of immune tolerance by genetic susceptibility (e.g., HLA alleles including HLA-DRB1*03:01, HLA-DQA1*05:01, HLA-A*03) and environmental triggers (e.g., viral infections, epigenetic modifications).Its core mechanism involves the activation of specific T/B lymphocytes, producing autoantibodies such as anti-SSA/Ro and anti-SSB/La. Immune cell infiltration damages exocrine glands including salivary and lacrimal glands, leading to reduced secretory function alongside systemic inflammatory responses and multi-organ involvement.

This embodiment provides four therapeutic target proteins for SS: RO60 (amino acid sequence as SEQ ID NO.160),TRIM21 (amino acid sequence as shown in SEQ ID NO.161), lupus La (amino acid sequence as shown in SEQ ID NO.162), and SPTN1 (amino acid sequence as shown in SEQ ID NO.163). The NetMHCipan_el 4.1 server was employed to predict peptide segments (epitopes) within these four target proteins capable of binding to MHC-II proteins.

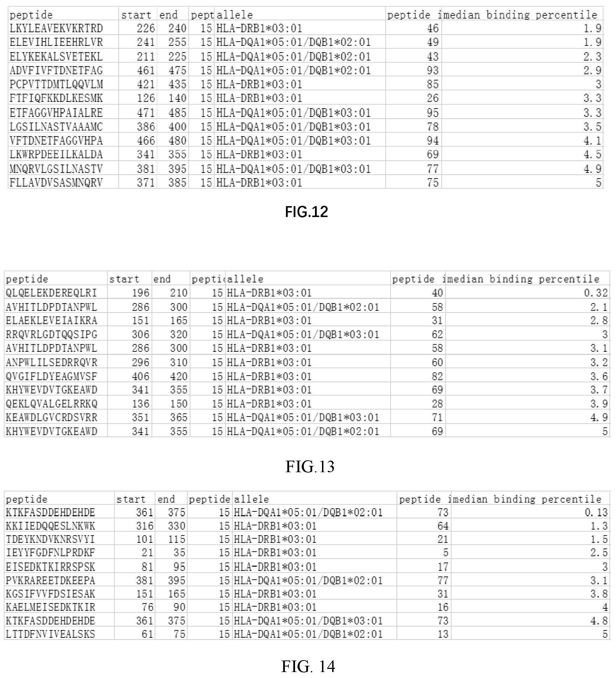

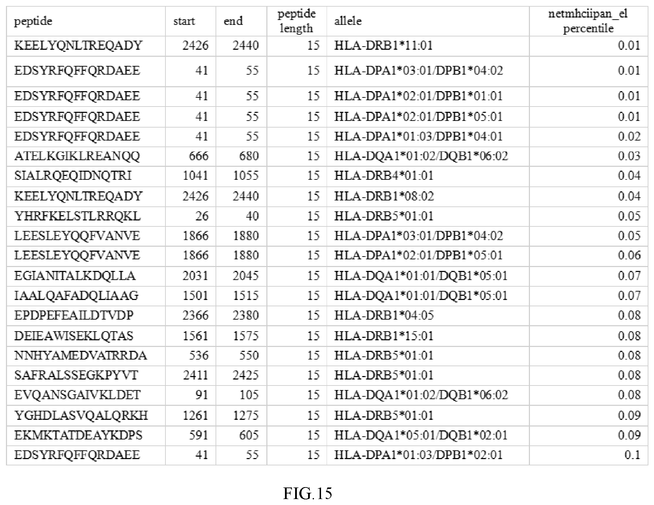

The binding patterns of epitopes in RO60, TRIM21, lupus La, and SPTN1 with HLA alleles are depicted in Figures 14-17 and . The preferred epitopes for the four target proteins, along with their flanking sequences selected from Figures 8-11, are summarised in Table 32 below.

**Table 32. Preferred epitopes and flanking sequences for four target proteins**

| Target Protein | Epitope Peptide | Start | Termination | Epitope Peptide with Flanking Sequence | SEQ ID NO. |
|---|---|---|---|---|---|
| | LKYLEAVEKVKRTRD | 226 | 240 | KLLKYLEAVEKVKRTRDEL | 164 |
| RO60 | ELEVIHLIEEHRLVR | 241 | 255 | RDELEVIHLIEEHRLVREH | 165 |
| | ELYKEKALSVETEKL | 211 | 225 | VHELYKEKALSVETEKLLK | 166 |
| TRIM21 | QLQELEKDEREQLRI | 196 | 210 | QRQLQELEKDEREQLRILG | 167 |
| | AVHITLDPDTANPWL | 286 | 300 | TCAVHITLDPDTANPWLIL | 168 |
| | ELAEKLEVEIAIKRA | 151 | 165 | KQELAEKLEVEIAIKRADW | 169 |
| lupus La | KTKFASDDEHDEHDE | 361 | 375 | GKKTKFASDDEHDEHDENG | 170 |
| | KKIIEDQQESLNKWK | 316 | 330 | ALKKIIEDQQESLNKWKSK | 171 |
| | TDEYKNDVKNRSVYI | 101 | 115 | EVTDEYKNDVKNRSVYIKG | 172 |
| SPTN1 | FQFFQRDAEELEKW | 46 | 59 | YRFQFFQRDAEELEKWIQ | 173 |

The reverse cDNA sequences corresponding to the peptides with flanking sequences in Table 32 are shown in Table 33 below.

**Table 33. Reverse cDNA sequences corresponding to peptides with flanking sequences**

| Target protein | Epitope Peptide with Flanking Sequences | Reverse cDNA sequence | SEQ ID NO. |
|---|---|---|---|
| RO60 | KLLKYLEAVEKVKRTRDEL | AAACTGCTGAAATATCTGGAAGCGGTGGAAAAAGTG AAACGCACCCGCGATGAACTG | 174 |
| | RDEVLIHIEEHRLVREH | CGCGATGAACTGGAAGTGATTCATCTGATTGAAGAACA TCGCCTGGTGCGCGAACAT | 175 |
| | VHELYKEKALSVETEKLLK | GTGCATGAACTGTATAAAGAAAAAGCGCTGAGCGTGGA AACCGAAAAACTGCTGAAA | 176 |
| TRIM21 | QRQLQELEKDEREQLRILG | CAGCGCCAGCTGCAGGAACTGGAAAAAGATGAAC GCGAACAGCTGCGCATTCTGGGC | 177 |
| | TCAVHITLDPDTANPWLIL | ACCTGCGCGGTGCATATTACCCTGGATCCGGATACCG CGAACCCGTGGCTGATTCTG | 178 |
| | KQELAEKLEVEIAIKRADW | AAACAGGAACTGGCGGAAAAACTGGAAGTGGAAATT GCGATTAAACGCGCGGATTGG | 179 |
| lupus La | GKKTKFASDDEHDEHDENG | GGCAAAAAAACCAAATTTGCGAGCGATGATGAACAT GATGAACATGATGAAAACGGC | 180 |
| | ALKKIIEDQQESLNKWKSK | GCGCTGAAAAAAATTATTGAAGATCAGCAGGAAA GCCTGAACAAATGGAAAAGCAAA | 181 |
| | EVTDEYKNDVKNRSVYIKG | GAAGTGACCGATGAATATAAAAACGATGTGAAAAAC CGCAGCGTGTATATTAAAGGC | 182 |
| SPTN1 | YRFQFFQRDAEELEKWIQ | TATCGCTTTCAGTTTTTCAGCGCGATGCGGAAGAACT GGAAAAATGGATTCAG | 183 |

Furthermore, a complete cDNA sequence is constructed, wherein the cDNA comprises a 5'UTR (including a Kozak sequence), a coding region, and a 3'UTR. The nucleotide sequences of the 5'UTR and 3'UTR are identical to those in Example 6. The coding region encodes the target sequence and at least one epitope sequence from Table 33, with multiple epitopes connected via linkers as part of the coding region. The leader sequence and linker sequence are as described in Example 6. In this example, the coding region of the cDNA sequence comprises all reverse-translated cDNA sequences from Table 33. The coding region cDNA undergoes further codon optimisation using the method described in Example 6. The optimised coding region cDNA sequence is shown in SEQ ID NO: 184, with a GC content of 57.03%.

Furthermore, the optimised cDNA is transcribed into mRNA using the method described in Example 6.

### 2. Preparation of LNPs

LNPs prepared using DSPC as the auxiliary lipid in Table 2 of Example 3, along with those from entries 1 and 7 (identical to Example 1), 22 and 26 in Table 4 of Example 5, were utilised.22 and 26 from Table 4 of Example 5 were loaded with the mRNA prepared in this example, designated as monovalent targeted LNPs, bivalent targeted LNPs, trivalent targeted LNPs, tetravalent targeted LNPs and pentavalent targeted LNPs respectively.

### 3. LNP Therapy for SS

The SS mouse model is established by breaking peripheral immune tolerance in susceptible strains through repeated subcutaneous immunisation with specific exocrine gland antigenic components or glandular extracts, supplemented with potent immunological adjuvants. Salivary gland extract (SG-extract) is the commonly employed antigen. During modelling, the antigen is typically mixed with complete Freund's adjuvant (CFA) for repeated subcutaneous immunisation. This induces chronic destructive inflammation targeting the lacrimal and salivary glands by activating effector T cells (such as Th1 and Th17 cells) while suppressing Treg activity. This model effectively mimics the characteristic lymphocytic infiltration around glands and subsequent decline in glandular secretory function observed in human SS.

SS mouse models were treated with intravenous injections of monovalent targeted LNPs, bivalent targeted LNPs, trivalent targeted LNPs, tetravalent targeted LNPs, and pentavalent targeted LNPs, administered every three weeks for a total of three doses. The control group received blank LNPs. Two weeks after the final injection, peripheral blood was collected to measure anti-SSA/Ro and anti-SSB/La antibody levels. Mice were then euthanised, and salivary glands were examined to determine the proportion of Foxp3⁺ Tregs and the levels of IL-17 and IFN-γ. The results are presented in Table 31 below.

**Table 34: Peripheral blood autoantibody levels, spleen Foxp3⁺ Treg proportion, central tissue IFN-γ, IL-17A and TNF-α levels, and nerve fibre damage scores in each group**

| Test item | Control | Monovalent Targeted LNPs | Bivalent-targeted LNPs | Trivalent Targeted LNPs | Quaternary Targeted LNPs | Pentavalent Targeted LNPs |
|---|---|---|---|---|---|---|
| Foxp3⁺ Tregs (%) | 5.2 | 9.4 | 14.1 | 17.3 | 11.8 | 10.2 |
| IFN-γ (pg/mg) | 43.5 | 32.6 | 19.8 | 7.8 | 23.5 | 28.7 |
| IL-17 (pg/mg) | 39.8 | 27.4 | 16.9 | 6.9 | 20.7 | 25.3 |
| Anti-SSA/Ro antibody (ng/mL) | 178.6 | 124.3 | 82.7 | 38.7 | 95.4 | 112.6 |
| Anti-SSB/La antibody (ng/mL) | 162.3 | 118.5 | 76.9 | 35.2 | 89.6 | 105.8 |

As shown in Table 34, compared with the control group, mice in the monovalent or multivalent targeted LNP groups exhibited reduced levels of autoantibodies against SSA/Ro and SSB/La, increased proportions of Foxp3⁺ Tregs, and decreased IFN-γ and IL-17A content. Among these, the trivalent targeted LNP group exhibited the lowest levels of both autoantibodies, the highest proportion of Foxp3⁺ Tregs, and the lowest IFN-γ and IL-17A levels. This indicates the strongest capacity to induce antigen-specific immune tolerance, with highly effective control of salivary gland and systemic inflammatory infiltration, thereby significantly reducing autoantibody-mediated exocrine gland damage.

The trivalent targeted LNPs synergistically targeted multiple receptors on the surface of LSECs. Simultaneous binding to multiple receptors significantly enhanced the specific uptake efficiency of LSECs. Following efficient uptake of LNPs by LSECs, on the one hand, they induced substantial proliferation of Foxp3⁺ Tregs, which secreted anti-inflammatory factors such as IL-10 to suppress the activation of reactive T/B cells; while simultaneously suppressing Th1/Th17 cell secretion of pro-inflammatory factors such as IFN-γ and IL-17. This reduces production of anti-SSA/Ro and anti-SSB/La antibodies, thereby blocking exocrine gland damage and systemic inflammatory responses to improve the pathological progression of SS.

Although the present invention has been disclosed as above, it is not limited thereto. Any person skilled in the art may make various changes and modifications without departing from the spirit and scope of the invention. Therefore, the scope of protection of the present invention shall be determined by the scope defined in the claims

## Claims

1. A lipid nanoparticle comprising cationically ionisable lipids; auxiliary lipids; ligand-modified PEGylated lipids; and cholesterol; wherein the ligand in the ligand-modified PEGylated lipid is capable of targeting receptors on the surface of hepatic sinusoidal endothelial cells, and the auxiliary lipid is selected from lipids capable of targeting receptors on the surface of hepatic sinusoidal endothelial cells or non-targeting lipids.

2. A lipid nanoparticle according to claim 1, wherein the cationically ionisable lipid companies any of SM102, MC3, and ALC-0315.

3. A lipid nanoparticle according to any one of claims1-2, wherein the auxiliary lipid comprises any one of PS, DOPS and DPPS.

4. A lipid nanoparticle according to any one of claims 1-3, wherein the ligand-modified PEGylated lipid comprises at least one of DSPE-PEG2K-mannose, DSPE-PEG2K-GlcNAc, DSPE-PEG2K-trimannose, DSPE-PEG2K-GalNAc-4-sulphate, DSPE-PEG2K-L-fucose, and DSPE-PEG2K-hyaluronic acid.

5. A lipid nanoparticle according to any one of claims 1-4, wherein the auxiliary lipid is PS; the ligand-modified PEGylated lipid is DSPE-PEG2K-mannose, DSPE-PEG2K-GlcNAc, DSPE-PEG2K-trimannose, DSPE-PEG2K-GalNAc-4-sulphate, DSPE-PEG2K-L-fucose and DSPE-PEG2K-hyaluronic acid.

6. The lipid nanoparticle according to any one of claims 1-5, wherein the ligand-modified PEGylated lipids are DSPE-PEG2K-trimannose and DSPE-PEG2K-GlcNAc.

7. The lipid nanoparticle according to any one of claims 1-6, wherein a molar ratio of cationically ionisable lipid, auxiliary lipid, ligand-modified PEGylated lipid and cholesterol is 20-70:1-15:1-5:25-45, with the sum of molar ratios of all components equalling 100%; wherein the N/P ratio of the cationically ionisable lipid is 2 to 6.

8. The lipid nanoparticle according to any one of claims 1-7, wherein the size of the lipid nanoparticle is 5 0 to 200 nm; the lipid nanoparticle encapsulates an RNA for treating allergic or autoimmune diseases.

9. The lipid nanoparticle according to claim 8, wherein the allergic diseases include birch pollen allergy and mugwort allergy, and the autoimmune diseases include type 1 diabetes, myasthenia gravis, toxic diffuse goitre, systemic lupus erythematosus, primary biliary cholangitis, multiple sclerosis and Sjögren's syndrome.

10. A lipid nanoparticle according to any one of claims 8-9, wherein the RNA encapsulated within the lipid nanoparticle for treating birch pollen allergy has a coding region encoding at least one amino acid sequence as shown in SEQ ID Nos. 2 to 7.

11. A lipid nanoparticle according to any one of claims 8-10, wherein the RNA encapsulated within the lipid nanoparticle for treating mugwort allergy has a coding region encoding at least one amino acid sequence as shown in SEQ ID Nos. 185-186; or wherein the RNA encapsulated within the lipid nanoparticle for treating type 1 diabetes has a coding region encoding at least one amino acid sequence as shown in SEQ ID Nos. 22-41;or wherein the RNA encapsulated within a lipid nanoparticle for treating the aforementioned myasthenia gravis, wherein the coding region encodes at least one amino acid sequence as shown in SEQ ID Nos. 64-69, 77-79;or wherein the RNA encapsulated within a lipid nanoparticle for treating the aforementioned toxic diffuse goitre, wherein the coding region encodes at least one amino acid sequence as shown in SEQ ID No. 86-93; or wherein the RNA encapsulated within the lipid nanoparticle for treating the systemic lupus erythematosus, wherein the coding region encodes at least one amino acid sequence as shown in SEQ ID No. 103-112; or wherein the RNA encapsulated within the lipid nanoparticle for treating the primary cholangitis, wherein the coding region encodes at least one amino acid sequence as shown in SEQ ID No. 123-131.

12. A lipid nanoparticle according to claim 8, wherein the RNA encapsulated within the lipid nanoparticles for treating the aforementioned multiple sclerosis, wherein the coding region encodes at least one amino acid sequence as shown in SEQ ID No. 143 to 150.

13. A lipid nanoparticle according to claim 8, wherein the RNA encapsulated within the lipid nanoparticles for treating the aforementioned Sjögren's syndrome, wherein the coding region encodes at least one amino acid sequence as shown in SEQ ID No. 164 to 173.
